# EUROPEAN PATENT APPLICATION

(11) **EP 4 667 489 A1**
(43) Date of publication of application: **24.12.2025**
(21) Application number: 24756297.8
(22) Date of filing: 08.02.2024
(51) Int. Cl.: C07K 16/00, A61K 39/395, C07K 16/46, C07K 16/28

(54) **POLYPEPTIDE AND ANTIBODY CONTAINING SAME ACTING AS LIGHT-CHAIN**

(30) Priority: 15.02.2023 CN 202310116909
(71) Applicant: Kyinno Biotechnology Co., Ltd., Beijing 101111 (CN)
(72) Inventor: NING, Jinying, Beijing 101111 (CN); PENG, Hao, Beijing 101111 (CN); HAO, Feng, Beijing 101111 (CN); WU, Guojin, Beijing 101111 (CN)
(74) Representative: Schiweck Weinzierl Koch Patentanwälte Partnerschaft mbB
(86) International application number: PCT/CN2024/076931
(87) International publication number: WO 2024/169925

(57) **Abstract**

Provided is a polypeptide, which is used as a light-chain or light-chain variable region of an antibody. The polypeptide is used to construct an antibody together with an antibody heavy-chain or antibody heavy-chain variable region having binding affinity to different targets (antigens), and the antibody retains binding affinity to targets (antigens) and biological activity. Further provided is an antibody containing the polypeptide.

## Description

### CROSS-REFERENCE TO RELATED APPLICATION(S)

The present patent application claims the benefit of priority to Chinese Patent Application No. CN202310116909.2 filed on 25 February 2023, which is incorporated herein by reference in its entirety for all purposes.

### TECHNICAL FIELD

The invention relates to the field of biomedicine, specifically to a peptide, its use as an antibody light chain, as well as a monoclonal antibody or a multispecific antibody comprising the peptide as a common light chain.

### BACKGROUND

Antibodies typically exist as one or more Y-shaped monomers, each composed of four polypeptide chains, i.e., two heavy chains and two light chains. The two heavy chains and two light chains of an antibody can be identical respectively, and such an antibody can be called a "homodimer", possessing binding affinity and specificity for one target, e.g., an antigen. Alternatively, the two heavy chains and two light chains of an antibody can be different respectively, and such an antibody can be called a "heterodimer", possessing binding affinity and specificity for two targets, e.g., antigens. Additional target-binding domains can also be incorporated into the Y-shaped monomer structure.

It is generally believed that the binding affinity and specificity of an antibody for a target, e.g., an antigen, are determined jointly by the six complementarity-determining regions (CDRs) in the paired heavy and light chains. However, it has been proposed to provide a polypeptide that can serve as a common light chain to pair with any heavy chain having binding affinity and specificity for a target, thereby constructing an antibody (homodimer). Building on this, the polypeptide can also serve as a common light chain to pair with two or more antibody heavy chains, further constructing bispecific or even multispecific antibodies (heterodimers, heteromultimers) with binding affinity and specificity for different targets.

Furthermore, currently conventional methods for preparing bispecific antibodies often require simultaneous transfection of four plasmids to express the two different heavy chains and two different light chains. However, during the assembly of these four polypeptide chains into an antibody, the problem of heavy-light chain mispairing can occur. Providing the aforementioned polypeptide as a common light chain pairing with two different heavy chains would avoid this problem.

### SUMMARY OF THE INVENTION

To address the above problems, an object of the present disclosure is to provide a polypeptide that, as an antibody light chain variable region, can be used to construct an antibody by pairing with an antibody heavy chain or heavy chain variable region specific for a certain target (antigen) or with two or more antibody heavy chains or heavy chain variable regions specific for different targets (antigens). Another object of the present disclosure is to provide an antibody comprising the polypeptide as a light chain variable region. Such an antibody may be, for example, in a homodimeric form or a heterodimeric form.

The present disclosure provides technical solutions as follows.

In a first aspect, the present disclosure provides a polypeptide, the amino acid sequence of which comprises:
(1) the amino acid sequence shown in SEQ ID NO. 25; the amino acid sequence shown in SEQ ID NO. 26; and the amino acid sequence shown in SEQ ID NO. 32;
(2) the amino acid sequence shown in SEQ ID NO. 31; the amino acid sequence shown in SEQ ID NO. 33; and the amino acid sequence shown in SEQ ID NO. 32; or
(3) the amino acid sequence shown in SEQ ID NO. 52; the amino acid sequence shown in SEQ ID NO. 26; and the amino acid sequence shown in SEQ ID NO. 53.

Further, the polypeptide may comprise the amino acid sequence shown in SEQ ID NO. 2, SEQ ID NO. 13, SEQ ID NO. 15, SEQ ID NO. 16, or SEQ ID NO. 41.
SEQ ID NO. 2: 
QIVLTQSPAIMSASPGEKVTMTCSASSSVSYMHWYQQKSGTSPKRWIYDTSKLASGVPAR
FSGSGSGTSYSLTISSMEAEDAATYYCQQWSSNPPYTFGSGTKLEIK
SEQ ID NO. 13: 
QIVLTQSPAIMSASPGEKVTMTCSATSGVSYMHWYQQKSGTSPKRWIYDTSRLASGVPAR
FSGSGSGTSYSLTISSMEAEDAATYYCQQWSSNPPYTFGSGTKLEIK
SEQ ID NO. 15: 
DIQITQSPSSLSASVGDRVTITCSATSGVSYMHWYQQKPGKAPKRLIYDTSRLASGVPSRF
SGSGSGTDYTLTISSLQPEDFATYYCQQWSSNPPYTFGQGTRLEIK
SEQ ID NO. 16: 
DIQITQSPSSLSASVGDRVTITCSASSSVSYMHWYQQKPGKAPKRLIYDTSKLASGVPSRFS
GSGSGTDYTLTISSLQPEDFATYYCQQWSSNPPYTFGQGTRLEIK
SEQ ID NO. 41:

DIQLTQSPSTLSASVGDRVTITCSATSSIGYMHWYQQKPGTSPKRWIYDTSKLASGVPSRF
SGSGSGTEYTLTISSLQPDDFATYYCQLWSSDPPYTFGQGTRLEIK

Further, the polypeptide may comprise the amino acid sequence shown in SEQ ID NO. 35, SEQ ID NO. 36, SEQ ID NO. 37, SEQ ID NO. 38, or SEQ ID NO. 43.
SEQ ID NO. 35: 
QIVLTQSPAIMSASPGEKVTMTCSASSSVSYMHWYQQKSGTSPKRWIYDTSKLASGVPAR
FSGSGSGTSYSLTISSMEAEDAATYYCQQWSSNPPYTFGSGTKLEIKRTVAAPSVFIFPPSD
EQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO. 36: 
QIVLTQSPAIMSASPGEKVTMTCSATSGVSYMHWYQQKSGTSPKRWIYDTSRLASGVPAR
FSGSGSGTSYSLTISSMEAEDAATYYCQQWSSNPPYTFGSGTKLEIKRTVAAPSVFIFPPSD
EQLKSGTASVVCLLNNFYPREAKVQWKVDNALQSGNSQESVTEQDSKDSTYSLSSTLTLS
KADYEKHKVYACEVTHQGLSSPVTKSFNRGEC
SEQ ID NO. 37: DIQITQSPSSLSASVGDRVTITCSATSGVSYMHWYQQKPGKAPKRLIYDTSRLASGVPSRF 
SGSGSGTDYTLTISSLQPEDFATYYCQQWSSNPPYTFGQGTRLEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC
SEQ ID NO. 38: 
DIQITQSPSSLSASVGDRVTITCSASSSVSYMHWYQQKPGKAPKRLIYDTSKLASGVPSRFS
GSGSGTDYTLTISSLQPEDFATYYCQQWSSNPPYTFGQGTRLEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC
SEQ ID NO. 43:

DIQLTQSPSTLSASVGDRVTITCSATSSIGYMHWYQQKPGTSPKRWIYDTSKLASGVPSRF
SGSGSGTEYTLTISSLQPDDFATYYCQLWSSDPPYTFGQGTRLEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

The polypeptide provided by the present disclosure, serving as an antibody light chain or antibody light chain variable region, can pair with antibody heavy chains or antibody heavy chain variable regions having binding affinity for different targets (antigens) to construct antibodies which possess the binding affinity for different targets (antigens). For example, the polypeptide, serving as an antibody light chain or antibody light chain variable region, can be used to pair with an antibody heavy chain or antibody heavy chain variable region having binding affinity for a certain target (antigen) to construct an antibody which possesses the binding affinity for the target (antigen); and the antibody is an antibody in homodimeric form (i.e., a monospecific antibody), having two identical heavy chains and two identical light chains. Alternatively, for example, the polypeptide, serving as an antibody light chain or antibody light chain variable region, can be used to pair with two antibody heavy chains or antibody heavy chain variable regions having binding affinity for two targets (antigens) respectively to construct an antibody which possesses the binding affinity for the two targets (antigens); and the antibody is an antibody in heterodimeric form (i.e., a bispecific antibody), having two different heavy chains and two identical light chains.

In a second aspect, the present disclosure provides an antibody or antigen-binding fragment thereof comprising the polypeptide provided in the first aspect of the present disclosure as an antibody light chain or antibody light chain variable region.

In the context of the present disclosure, the antigen-binding fragment refers to any functional fragment of an antibody capable of specifically binding a target. In the context of the present disclosure, terms "target" and "antigen" are used interchangeably.

The antibody or antigen-binding fragment thereof provided by the present disclosure may be an IgG-like antibody. In the present disclosure, an "IgG-like antibody" means that the antibody or antigen-binding fragment thereof has at least two Fab arms and an optional Fc region, being of the traditional monoclonal antibody (mAb) structure or a similar structure. Therefore, in its at least one Fab arm, the antibody or antigen-binding fragment thereof provided by the present disclosure may comprise the amino acid sequences defined in any one of (1) to (3) of the first aspect of the present disclosure as light chain CDRs; or may comprise the amino acid sequence shown in SEQ ID NO. 2, SEQ ID NO. 13, SEQ ID NO. 15, SEQ ID NO. 16, or SEQ ID NO. 41 as a light chain variable region (VL). Alternatively, the antibody or antigen-binding fragment thereof provided by the present disclosure may comprise the amino acid sequence shown in SEQ ID NO. 35, SEQ ID NO. 36, SEQ ID NO. 37, SEQ ID NO. 38, or SEQ ID NO. 43 as a light chain.

Further, in its at least one Fab arm, the antibody or antigen-binding fragment thereof may further comprise a heavy chain variable region (VH) which forms a binding domain for a target with the light chain variable region. According to specific embodiments of the invention, the target can be programmed death factor 1 (PD-1), lymphocyte activation gene 3 (LAG-3), programmed cell death 1 ligand 1 (PDL1) or epidermal growth factor receptor (EGFR).

According to specific embodiments of the invention, in its at least one Fab arm, the antibody or antigen-binding fragment thereof may comprise heavy chain CDRs (HCDR1, HCDR2, and HCDR3) and light chain CDRs (LCDR1, LCDR2, and LCDR3) respectively as follows:
(1) an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(2) an HCDR1 comprising the amino acid sequence shown in SEQ ID NO. 22, an HCDR2 comprising the amino acid sequence shown in SEQ ID NO. 23, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(3) an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(4) an HCDR1 comprising the amino acid sequence shown in SEQ ID NO. 22, an HCDR2 comprising the amino acid sequence shown in SEQ ID NO. 23, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(5) an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(6) an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(7) an HCDR1 comprising the amino acid sequence shown in SEQ ID NO. 48, an HCDR2 comprising the amino acid sequence shown in SEQ ID NO. 49, and an HCDR3 comprising the amino acid sequence shown in SEQ ID NO. 50; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(8) an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53;
(9) an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(10) an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; or
(11) an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53.

Further, in its at least one Fab arm, the antibody or antigen-binding fragment thereof may comprise a heavy chain variable region and a light chain variable region comprising respectively:
(1) the amino acid sequence shown in SEQ ID NO. 12; and, the amino acid sequence shown in SEQ ID NO. 2;
(2) the amino acid sequence shown in SEQ ID NO. 10; and, the amino acid sequence shown in SEQ ID NO. 2;
(3) the amino acid sequence shown in SEQ ID NO. 12; and, the amino acid sequence shown in SEQ ID NO. 13;
(4) the amino acid sequence shown in SEQ ID NO. 10; and, the amino acid sequence shown in SEQ ID NO. 13;
(5) the amino acid sequence shown in SEQ ID NO. 14; and, the amino acid sequence shown in SEQ ID NO. 15;
(6) the amino acid sequence shown in SEQ ID NO. 19; and, the amino acid sequence shown in SEQ ID NO. 15;
(7) the amino acid sequence shown in SEQ ID NO. 14; and, the amino acid sequence shown in SEQ ID NO. 16;
(8) the amino acid sequence shown in SEQ ID NO. 19; and, the amino acid sequence shown in SEQ ID NO. 16;
(9) the amino acid sequence shown in SEQ ID NO. 39; and, the amino acid sequence shown in SEQ ID NO. 2;
(10) the amino acid sequence shown in SEQ ID NO. 40; and, the amino acid sequence shown in SEQ ID NO. 41;
(11) the amino acid sequence shown in SEQ ID NO. 44; and, the amino acid sequence shown in SEQ ID NO. 13;
(12) the amino acid sequence shown in SEQ ID NO. 45; and, the amino acid sequence shown in SEQ ID NO. 15;
(13) the amino acid sequence shown in SEQ ID NO. 40; and, the amino acid sequence shown in SEQ ID NO. 15; or
(14) the amino acid sequence shown in SEQ ID NO. 45; and, the amino acid sequence shown in SEQ ID NO. 41.

The antibody or antigen-binding fragment thereof may be a murine antibody, a chimeric antibody or a humanized antibody; and may further comprise a heavy chain constant region and a light chain constant region. According to specific embodiments of the invention, the heavy chain constant region may be a heavy chain constant region of IgG1 or IgG4; and the light chain constant region may be a kappa or lambda light chain constant region. For example, the heavy chain constant region of the antibody may comprise the amino acid sequence shown in SEQ ID NO. 17 (hIgG4) or SEQ ID NO. 42 (hIgG1); and the light chain constant region of the antibody may comprise the amino acid sequence shown in SEQ ID NO. 18 (hkappa). Alternatively, the heavy chain constant region of the antibody may comprise the amino acid sequence shown in SEQ ID NO. 20 (hIgG4; Knob) or SEQ ID NO. 21 (hIgG4; Hole); and the light chain constant region of the antibody may comprise the amino acid sequence shown in SEQ ID NO. 18. Alternatively, the heavy chain constant region of the antibody may comprise the amino acid sequence shown in SEQ ID NO. 46 (hIgG1; Knob) or SEQ ID NO. 47 (hIgG1; Hole); and the light chain constant region of the antibody may comprise the amino acid sequence shown in SEQ ID NO. 18.

The at least two Fab arms of the antibody or antigen-binding fragment thereof provided by the present disclosure may be identical, thereby binding the same target (antigen). In this case, in its at least two Fab arms, the antibody or antigen-binding fragment thereof may comprise identical amino acid sequence combination selected from any one of (1) to (11) provided above for HCDR1 to HCDR3 and LCDR1 to LCDR3 or comprise identical amino acid sequence combination selected from any one of (1) to (14) provided above for heavy chain and light chain variable regions. The antibody or antigen-binding fragment thereof may further comprise a heavy chain constant region and a light chain constant region. As described above, for example, the heavy chain constant region may comprise the amino acid sequence shown in SEQ ID NO. 17 or SEQ ID NO. 42; and the light chain constant region may comprise the amino acid sequence shown in SEQ ID NO. 18. Further, the antibody or antigen-binding fragment thereof may have a heavy chain and a light chain, for example, being a monoclonal antibody which has two identical heavy chains and two identical light chains.

Alternatively, the at least two Fab arms of the antibody or antigen-binding fragment thereof provided by the present disclosure may be different, thereby binding different targets (antigens). In this case, in its at least two Fab arms, the antibody or antigen-binding fragment thereof may comprise different amino acid sequence combinations selected from any one of (1) to (11) provided above for HCDR1 to HCDR3 and LCDR1 to LCDR3 or comprise different amino acid sequence combinations selected from any one of (1) to (14) provided above for heavy chain and light chain variable regions. In this case, it is preferable that the at least two Fab arms of the antibody or antigen-binding fragment thereof comprise identical light chain CDRs sequences or comprise identical light chain variable sequences. For example, the at least two Fab arms both comprise: (1) an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32; (2) an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; or (3) an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53. Further, for example, the at least two Fab arms both comprise: (1) a light chain variable region comprising the amino acid sequence shown in SEQ ID NO. 2; (2) a light chain variable region comprising the amino acid sequence shown in SEQ ID NO. 13; (3) a light chain variable region comprising the amino acid sequence shown in SEQ ID NO. 15; (4) a light chain variable region comprising the amino acid sequence shown in SEQ ID NO. 16; or (5) a light chain variable region comprising the amino acid sequence shown in SEQ ID NO. 41.

According to specific embodiments of the invention, the antibody or antigen-binding fragment thereof may comprise:
(1) in one Fab arm: an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; and
   in another Fab arm: an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(2) in one Fab arm: an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32; and
   in another Fab arm: an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(3) in one Fab arm: an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; and
   in another Fab arm: an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; or
(4) in one Fab arm: an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53; and
   in another Fab arm: an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53.

According to specific embodiments of the invention, further, the antibody or antigen-binding fragment thereof may comprise:
(1) in one Fab arm: a heavy chain variable region comprising the amino acid sequence shown in SEQ ID NO. 14, and a light chain variable region comprising the amino acid sequence shown in SEQ ID NO. 15; and
   in another Fab arm: a heavy chain variable region comprising the amino acid sequence shown in SEQ ID NO. 19, and a light chain variable region comprising the amino acid sequence shown in SEQ ID NO. 15;
(2) in one Fab arm: a heavy chain variable region comprising the amino acid sequence shown in SEQ ID NO. 14, and a light chain variable region comprising the amino acid sequence shown in SEQ ID NO. 16; and
   in another Fab arm: a heavy chain variable region comprising the amino acid sequence shown in SEQ ID NO. 19, and a light chain variable region comprising the amino acid sequence shown in SEQ ID NO. 16;
(3) in one Fab arm: a heavy chain variable region comprising the amino acid sequence shown in SEQ ID NO. 45, and a light chain variable region comprising the amino acid sequence shown in SEQ ID NO. 15; and
   in another Fab arm: a heavy chain variable region comprising the amino acid sequence shown in SEQ ID NO. 40, and a light chain variable region comprising the amino acid sequence shown in SEQ ID NO. 15; or
(4) in one Fab arm: a heavy chain variable region comprising the amino acid sequence shown in SEQ ID NO. 45, and a light chain variable region comprising the amino acid sequence shown in SEQ ID NO. 41; and
in another Fab arm: a heavy chain variable region comprising the amino acid sequence shown in SEQ ID NO. 40, and a light chain variable region comprising the amino acid sequence shown in SEQ ID NO. 41.

In this case, the antibody or antigen-binding fragment thereof may also comprise a heavy chain constant region and a light chain constant region. For example, the heavy chain constant region of the antibody may comprise the amino acid sequence shown in SEQ ID NO. 20 or SEQ ID NO. 21; and the light chain constant region of the antibody may comprise the amino acid sequence shown in SEQ ID NO. 18. Alternatively, the heavy chain constant region of the antibody may comprise the amino acid sequence shown in SEQ ID NO. 46 (hIgG1; Knob) or SEQ ID NO. 47 (hIgG1; Hole); and the light chain constant region of the antibody may comprise the amino acid sequence shown in SEQ ID NO. 18.

Further, the antibody may be a bispecific antibody having two different heavy chains but comprising two identical light chains. According to specific embodiments of the invention, the bispecific antibody may comprise the amino acid sequence shown in SEQ ID NO. 35, SEQ ID NO. 36, SEQ ID NO. 37, SEQ ID NO. 38 or SEQ ID NO. 43 as a light chain.

According to specific embodiments of the invention, the bispecific antibodies provided by the present disclosure are shown in Table 9 and Table 36 in the part of "DETAILED DESCRIPTION OF PREFERRED EMBODIMENTS".

The bispecific antibodies shown in Table 9 include the following domains:
Bispecific antibody KA-1835 H28G27V4×H107D1210-hIgG4M and KA-1836 H107D1210×H28G27V4-hIgG4M, both comprising two Fab arms targeting PD1 and LAG3 respectively, in which one Fab arm comprises a heavy chain variable region targeting PD1 (SEQ ID NO. 14) paired with a light chain variable region (SEQ ID NO. 15), and the other Fab arm comprises a heavy chain variable region targeting LAG3 (SEQ ID NO. 19) paired with a light chain variable region (SEQ ID NO. 15). This bispecific antibody comprises two identical light chains (SEQ ID NO. 37).
Bispecific antibody KA-1793 H28G27V4×H107D1210-hIgG4M, comprising two Fab arms targeting PD1 and LAG3 respectively, in which one Fab arm comprises a heavy chain variable region targeting PD1 (SEQ ID NO. 14) paired with a light chain variable region (SEQ ID NO. 16), and the other Fab arm comprises a heavy chain variable region targeting LAG3 (SEQ ID NO. 19) paired with a light chain variable region (SEQ ID NO. 16). This bispecific antibody comprises two identical light chains (SEQ ID NO. 38).

The bispecific antibodies shown in Table 36 include the following domains:
Bispecific antibody KA-2072 H22A12×58E3-CLC01, comprising two Fab arms targeting EGFR and PDL1 respectively, in which one Fab arm comprises a heavy chain variable region targeting EGFR (SEQ ID NO. 45) paired with a light chain variable region (SEQ ID NO. 15), and the other Fab arm comprises a heavy chain variable region targeting PDL1 (SEQ ID NO. 40) paired with a light chain variable region (SEQ ID NO. 15). This bispecific antibody comprises two identical light chains (SEQ ID NO. 37).
Bispecific antibody KA-2073 H22A12×58E3-CLC02, comprising two Fab arms targeting EGFR and PDL1 respectively, in which one Fab arm comprises a heavy chain variable region targeting EGFR (SEQ ID NO. 45) paired with a light chain variable region (SEQ ID NO. 41), and the other Fab arm comprises a heavy chain variable region targeting PDL1 (SEQ ID NO. 40) paired with a light chain variable region (SEQ ID NO. 41). This bispecific antibody comprises two identical light chains (SEQ ID NO. 43).

The present disclosure provides a polypeptide. Experiments proved that the polypeptide, serving as an antibody light chain or antibody light chain variable region, can pair with antibody heavy chains or antibody heavy chain variable regions having binding affinity for different targets (antigens) to construct antibodies which possess the binding affinity for different targets (antigens) and biological activities.

In a third aspect, the present disclosure provides use of the polypeptide described in the first aspect in the construction of an antibody. As described above, the antibody may be an antibody in homodimeric form with two identical heavy chains and two identical light chains, or an antibody in heterodimeric form with two different heavy chains and two identical light chains. The polypeptide can be used as the light chain or light chain variable region of the antibody.

In a fourth aspect, the present disclosure provides a nucleic acid molecule comprising a nucleotide sequence encoding the polypeptide described in the first aspect.

### BRIEF DESCRIPTION OF THE DRAWINGS

Embodiments of the invention are described in detail below with reference to the attached figures, in which:
Figure 1: Detection results of the binding of hybridoma cell culture supernatants containing anti-LAG3 antibodies to LAG3-expressing cells;
Figure 2: Detection results of the blocking of LAG3 binding to tumor cells by hybridoma cell culture supernatants containing anti-LAG3 antibodies;
Figure 3: Detection results of the binding of hybridoma cell culture supernatants containing anti-PD1 antibodies to PD1-expressing cells;
Figure 4: Detection results of the blocking of PD1 binding to PDL1-expressing cells by hybridoma cell culture supernatants containing anti-PD1 antibodies;
Figure 5: Detection results of the binding of anti-PD1 humanized antibodies and chimeric antibodies to PD1-expressing cells;
Figure 6: Detection results of the blocking of PD1 binding to PDL1-expressing cells by anti-PD1 humanized antibodies and chimeric antibodies;
Figure 7: Detection results of the binding of anti-LAG3 humanized antibodies and chimeric antibodies to LAG3-expressing cells;
Figure 8: Detection results of the blocking of LAG3 binding to tumor cells by anti-LAG3 humanized antibodies and chimeric antibodies;
Figure 9: Detection results of the binding of PD1×LAG3 bispecific antibodies to PD1-expressing cells;
Figure 10: Detection results of the binding of PD1×LAG3 bispecific antibodies to LAG3-expressing cells;
Figure 11: Detection results of the blocking of PD1 binding to PDL1-expressing cells by PD1×LAG3 bispecific antibodies;
Figure 12: Detection results of the blocking of LAG3 binding to tumor cells by PD1×LAG3 bispecific antibodies;
Figure 13: Detection results of the binding of PD1×LAG3 bispecific antibodies to PD1-expressing cells;
Figure 14: Detection results of the binding of PD1×LAG3 bispecific antibodies to LAG3-expressing cells;
Figure 15: Detection results of the blocking of PD1 binding to PDL1-expressing cells by PD1 ×LAG3 bispecific antibodies;
Figure 16: Detection results of the blocking of LAG3 binding to tumor cells by PD1×LAG3 bispecific antibodies;
Figure 17: Detection results of the binding of hybridoma cell culture supernatants containing anti-PDL1 antibodies to PDL1-expressing cells, in which 17A: positive control antibody, 17B: hybridoma cell culture supernatants;
Figure 18: Detection results of the blocking of PDL1 binding to PD1-expressing cells by hybridoma cell culture supernatants containing anti-PDL1 antibodies, in which 18A: positive control antibody, 18B: hybridoma cell culture supernatants;
Figure 19: Detection results of the blocking of PDL1/PD1 signaling by anti-PDL1 murine antibodies;
Figure 20: Detection results of the binding of anti-PDL1 humanized antibodies to PDL1-expressing cells;
Figure 21: Detection results of the blocking of PDL1 binding to PD1-expressing cells by anti-PDL1 humanized antibodies;
Figure 22: Detection results of the blocking of PDL1/PD1 signaling by anti-PDL1 humanized antibodies;
Figure 23: Detection results of the binding of hybridoma cell culture supernatants containing anti-EGFR antibodies to EGFR-expressing cells, in which 23A: positive control antibody, 23B: hybridoma cell culture supernatants;
Figure 24: Detection results of the blocking of EGFR-Fc binding to EGF by anti-EGFR murine antibodies, in which 24A: positive control antibody, 24B: anti-EGFR murine antibodies;
Figure 25: Detection results of the blocking of EGFR signaling by anti-EGFR murine antibodies;
Figure 26: Detection results of the binding of anti-EGFR humanized antibodies to EGFR-expressing cells;
Figure 27: Detection results of the blocking of EGFR-Fc binding to EGF by anti-EGFR humanized antibodies;
Figure 28: Detection results of the blocking of EGFR signaling by anti-EGFR humanized antibodies;
Figure 29: Detection results of the binding of EGFR×PDL1 bispecific antibodies to PDL1-expressing cells;
Figure 30: Detection results of the blocking of PDL1 binding to PD1-expressing cells by EGFR×PDL1 bispecific antibodies;
Figure 31: Detection results of the blocking of PDL1/PD1 signaling by EGFR×PDL1 bispecific antibodies;
Figure 32: Detection results of the binding of EGFR×PDL1 bispecific antibodies to EGFR-expressing cells;
Figure 33: Detection results of the blocking of EGF binding to EGFR-expressing cells by EGFR×PDL1 bispecific antibodies; and
Figure 34: Detection results of the blocking of EGFR signaling by EGFR×PDL1 bispecific antibodies.

### DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

### Example 1 Screening for common light chain

Prior art analysis revealed that germline sequences like KV4 exhibit higher expression frequencies in mice and pair with mouse heavy chains at higher rates. Therefore, this category of germline sequences was selected, from which the following mouse germline sequence KV4-59, which is relatively easy to humanize, and has good solubility, low immunogenicity and no potential modification sites, was chosen.
> KV4-59 (SEQ ID NO. 1):

QIVLTQSPAIMSASPGEKVTMTCSASSSVSYMHWYQQKSGTSPKRWIYDTSKLASGVPARFSGSGS

GTSYSLTISSMEAEDAATYYCQQWSSNPPFGSGTKLEIK

The sequence was modified. Since the light chain CDR3 plays an important role in the affinity of antibody-antigen binding, amino acids were added after the CDR3 (see the underlined) to improve affinity for potential antigens. The following three sequences were obtained:
> KV4-59-1 (SEQ ID NO. 2): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIK
> **KV4-59-2** (SEQ ID NO. 3): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPWTFGSGTKLEIK
> KV4-59-3 (SEQ ID NO. 4):

QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPFTFGSGTKLEIK

The above three sequences were used as antibody light chain variable region sequences respectively and paired with the heavy chain variable region sequence of a murine anti-chicken lysozyme antibody as follows to construct chimeric antibodies further using human heavy and light chain constant regions.
> Heavy chain variable region sequence of murine anti-chicken lysozyme antibody (SEQ ID NO. 5):

LDVKLQESGPGLVKPSQSLSLTCSVTGYSFTSGYYWNWIRLFPGNKLEWMGYVSYFGTNNYNPSL
KNRFSITRDTSKNQFFLKLNSVSTEDTATYYCARGEEWDWSFDVWGTGTTVTVSS

By detecting properties e.g., expression level, thermal stability, solubility, and freeze-thaw stability of the three chimeric antibodies, it was confirmed that when KV4-59-1 (SEQ ID NO. 2) was used as the antibody light chain variable region sequence, the constructed antibody had superior properties.

### Example 2 Construction of transgenic mice

Based on the antibody lambda light chain gene sequences of C57 mice, sgRNAs were designed from both ends of the gene cluster. Using the Cas9-gRNA Target Efficiency Detection Kit (Vazyme, Cat No.: VK007-30T), the following sgRNA sequences exhibiting high cleavage efficiency were selected through in vitro validation: TGAATGCCATGTACTTATGG (SEQ ID NO. 6); and AAGTTCAGCTCCTAAAATGG (SEQ ID NO. 7). The sgRNA sequences were synthesized and microinjected into mouse fertilized eggs along with spCas91.1 protein. The sgRNAs mediated the Cas9 protein to cleave at both ends of the fragment of target gene to be deleted, causing DNA double-strand breaks. The genomic ends were repaired via the non-homologous end joining (NHEJ) pathway, resulting in deletion of the middle target fragment and achieving knock-out of the target gene. Two weeks after birth, mice were genotyped to confirm lambda gene knock-out.

Based on the antibody kappa light chain sequences of mice, sgRNAs were designed. Using the Cas9-gRNA Target Efficiency Detection Kit (Vazyme, Cat No.: VK007-30T), the following sgRNA sequences exhibiting high cleavage efficiency were selected through in vitro validation: IGK-L1: GTGAATGCCATGTACTTATGG (SEQ ID NO. 8); IGK-R1: CAAGTTCAGCTCCTAAAATGG (SEQ ID NO. 9). The sgRNA sequences were synthesized and microinjected into mouse fertilized eggs along with spCas91.1 protein. The sgRNAs mediated the Cas9 protein to cleave at the target position, causing a nick. Using a Donor DNA fragment with homologous arms and the target fragment KV4-59-1 (SEQ ID NO. 2), DNA fragment knock-in was achieved at the target position via homologous recombination. Two weeks after birth, mice were genotyped to confirm common light chain gene knock-in.

The heterozygous mice with lambda chain knock-out were bred with the heterozygous mice carrying the common light chain knock-in. Two weeks after birth, each offspring underwent genotyping to detect the presence of the common light chain knock-in and the status of lambda chain knock-out. This process continued until mice that were homozygous for the common light chain knock-in and both lambda and kappa chains knock-out were obtained.

### Example 3 Preparation of hybridoma cells secreting anti-LAG3 antibodies

Mice constructed in Example 2 were immunized with a fusion protein (referred to as "LAG3-mFc") of the human LAG3 extracellular domain (NCBI Reference Sequence: NP_002277.4) and mouse IgG2a-Fc (Genbank Accession No. AAH31470.1). Mice with higher titers were selected, from which sera were collected. Then the mice were dissected, their spleens were taken and spleen cells were isolated. The spleen cells were fused with cultured myeloma cells, and hybridoma cells were obtained.

ELISA plates were coated with a fusion protein (referred to as "LAG3-hFc") of the human LAG3 extracellular domain and human IgG1-Fc (Genbank Accession No. CAC20454.1). The binding activity of hybridoma cell culture supernatants to this fusion protein was detected by ELISA, yielding multiple positive hybridoma cell lines secreting anti-LAG3 antibodies.

### Example 4 Detection of the binding of hybridoma cell culture supernatants containing anti-LAG3 antibodies to LAG3-expressing cells

The gene sequence encoding human LAG3 (NCBI Reference Sequence: NM_002286.6) was cloned into the PLVX packaging vector (Clontech, virus package mix, Cat No.: 631275), and the recombinant plasmid was transfected into 293T cells; then, resistant cell lines were selected with puromycin, obtaining 293T cells stably expressing human LAG3 (referred to as "293T-LAG3 cells"; Kyinno Biotechnology Co. Ltd. (referred to as "Kyinno"), Cat No.: KC-0214). The 293T-LAG3 cells were then prepared as a cell suspension at a concentration of 1×10⁷ cells/mL in PBS containing 2% FBS.

50 µL of the cell suspension was added into one of flow cytometry tubes (sample tubes), then 50 µL of either the hybridoma cell culture supernatant to be detected or control antibody 28F6-7 was added, followed by 60 minutes of incubation at 4 °C. Next, 1 mL of flow cytometry buffer was added to each flow cytometry tube, which was then centrifuged at 1200 rpm for 5 minutes, and supernatant was discarded. This wash step was repeated three times. Simultaneously, control tube 1 (only cell suspension added, but no culture supernatant and secondary antibody (described below)); and control tube 2 (only cell suspension and secondary antibody added, but no culture supernatant) were set.

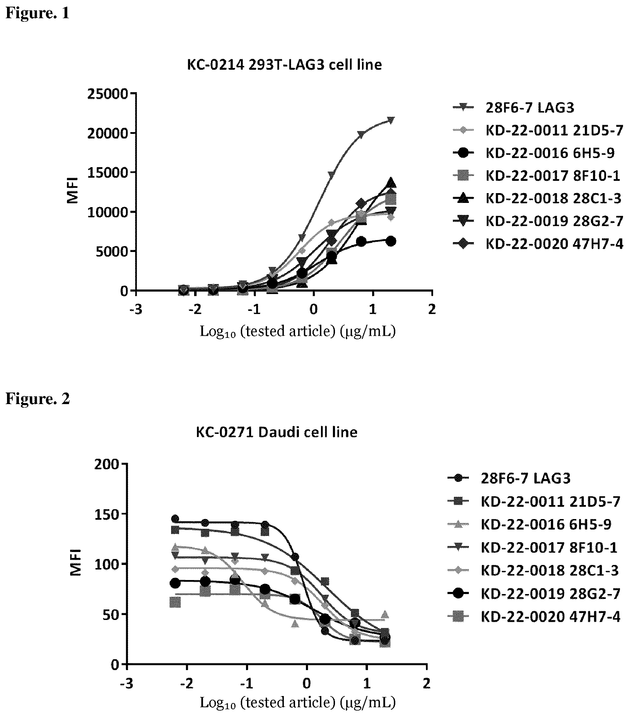

100 µL of flow cytometry buffer was added to each flow cytometry tube to re-suspend the cells, and then 5 µL of PE-conjugated anti-mouse IgG Fc secondary antibody (PE anti-mouse IgG Fc; Biolegend, Cat No.: 409304) was added, followed by 30 minutes of incubation at 4 °C protected from light. Next, 1 mL of flow cytometry buffer was added to each tube, which was then centrifuged at 1200 rpm at room temperature for 5 minutes and supernatant was discarded. This wash step was repeated three times. 250 µL of flow cytometry buffer was added to each flow cytometry tube to re-suspend the cells again, and the cells were mixed thoroughly and detected. Detection results are shown in Table 1 and Figure 1.

**Table 1. Detection results of the binding of hybridoma cell culture supernatants containing anti-LAG3 antibodies to LAG3-expressing cells**

| Hybridoma cell line | 28F6-7 | KD-22-0011 21D5-7 | KD-22-0016 6H5-9 | KD-22-0017 8F10-1 | KD-22-0018 28C1-3 | KD-0019 28G2-7 | KD-22-0020 47H7-4 |
|---|---|---|---|---|---|---|---|
| EC50 (µg/mL) | 1.228 | 0.5838 | 1.138 | 2.849 | 5.410 | 1.081 | 1.979 |
| MFI.Max | 21523 | 9831 | 6275 | 11549 | 13703 | 9986 | 12300 |

| | | | | | | | |
|---|---|---|---|---|---|---|---|
| Note: control antibody 28F6-7 is a previously validated anti-LAG3 hybridoma antibody. | | | | | | | |

### Example 5 Detection of the blocking of LAG3 binding to tumor cells by hybridoma cell culture supernatants containing anti-LAG3 antibodies

Daudi cells (Kyinno, Cat No.: KC-0271) were collected, washed once with FACS buffer, and plated at 2-5×10⁵ cells/well in 96-deep well plates. 50 µL of hybridoma cell culture supernatant or control antibody 28F6-7 (3.16-fold serially diluted from a starting concentration of 20 µg/mL) and 500 ng of the fusion protein LAG3-hFc were added per well as a premix into the plates, followed by 2 hours of incubation. Next, 400 µL of FACS buffer was added per well to wash twice, and then PE-conjugated anti-human IgG Fc secondary antibody (PE anti-human IgG Fc; SouthernBiotech, Cat No.: 2010-09) was added, followed by 1 hour of incubation. 400 µL of FACS buffer was added per well to wash twice again, then the cells were detected. Detection results are shown in Table 2 and Figure 2.

**Table 2. Detection results of the blocking of LAG3 binding to tumor cells by hybridoma cell culture supernatants containing anti-LAG3 antibodies**

| Hybridoma cell line | 28F6-7 | KD-22-0011 21D5-7 | KD-22-0016 6H5-9 | KD-22-0017 8F10-1 | KD-22-0018 28C1-3 | KD-0019 28G2-7 | KD-22-0020 47H7-4 | mIgG irrelevant antibody |
|---|---|---|---|---|---|---|---|---|
| IC50 (µg/mL) | 0.8656 | 2.187 | 0.08788 | 1.775 | 1.846 | 1.263 | 1.735 | N/A |

A murine antibody was obtained from the hybridoma cell line KD-0019 28G2-7 and was named "28G2". Its variable region sequences are as follows (heavy and light chain CDRs are underlined and identified according to the KABAT numbering scheme; *Ibid. for subsequent cases):*
> Heavy chain variable region of anti-LAG3 murine antibody 28G2 (SEQ ID NO. 10; HCDRs: SEQ ID NOs. 22/23/24): 
DVKLQESGPGLVKPSQSLSLTCSVTGYSFTSGYYWNWIRLFPGNKLEWMGYVSYFGTNNYNPSL
KNRFSITRDTSKNQFFLKLNSVSTEDTATYYCARGEEWDWSFDVWGTGTTVTVSS
> Light chain variable region of anti-LAG3 murine antibody 28G2 (SEQ ID NO. 11; LCDRs: SEQ ID NOs. 25/26/27):

DIVLTQSPAFMSASPGEKVTMTCSASSSVSYMHWYQQKSGTSPKRWIYDTSKLASGVPTRFSGSGS
GTSYSLTISSMEAEDAATYYCQQWSTNPPYTFGGGTKLEIK

### Example 6 Preparation of hybridoma cells secreting anti-PD1 antibodies

Mice constructed in Example 2 were immunized with a fusion protein (referred to as "PD1-mFc") of the human PD1 extracellular domain (NCBI Reference Sequence: NP_005009.2) and mouse IgG2a-Fc (Genbank Accession No. AAH31470.1). Mice with higher titers were selected, from which sera were collected. Then the mice were dissected, their spleens were taken and spleen cells were isolated. The spleen cells were fused with cultured myeloma cells, and hybridoma cells were obtained.

ELISA plates were coated with a fusion protein (referred to as "PD1-hFc") of the human PD1 extracellular domain and human IgG1-Fc (Genbank Accession No. CAC20454.1). The binding activity of hybridoma cell culture supernatants to this fusion protein was detected by ELISA, yielding multiple positive hybridoma cell lines secreting anti-PD1 antibodies.

### Example 7 Detection of the binding of hybridoma cell culture supernatants containing anti-PD1 antibodies to PD1-expressing cells

The gene sequence encoding human PD1 (NCBI Reference Sequence: NM_005018.3) was cloned into the PLVX packaging vector (Clontech, virus package mix, Cat No.: 631275), and the recombinant plasmid was transfected into 293T cells; then, resistant cell lines were selected with puromycin, obtaining 293T cells stably expressing human PD1 (referred to as "293T-PD1 cells"; Kyinno, Cat No.: KC-0204). The 293T-PD1 cells were then prepared as a cell suspension at a concentration of 1×10⁷ cells/mL in PBS containing 2% FBS.

50 µL of the cell suspension was added into one of flow cytometry tubes (sample tubes), then 50 µL of the hybridoma cell culture supernatant to be detected was added, followed by 60 minutes of incubation at 4 °C. Next, 1 mL of flow cytometry buffer was added to each flow cytometry tube, which was then centrifuged at 1200 rpm for 5 minutes, and supernatant was discarded. This wash step was repeated three times. Simultaneously, control tube 1 (only cell suspension added, but no culture supernatant and secondary antibody (described below)); and control tube 2 (only cell suspension and secondary antibody added, but no culture supernatant) were set.

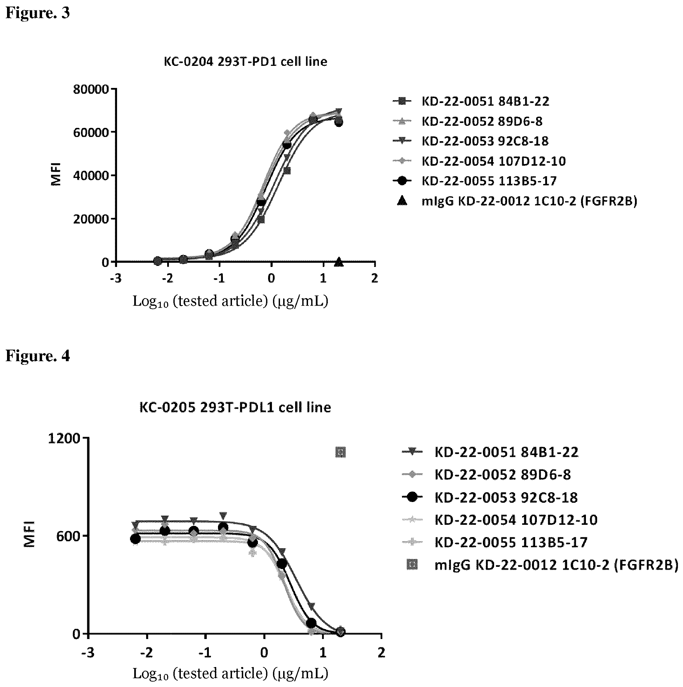

100 µL of flow cytometry buffer was added to each flow cytometry tube to re-suspend the cells, and then 5 µL of PE-conjugated anti-mouse IgG Fc secondary antibody (Biolegend, Cat No.: 409304) was added, followed by 30 minutes of incubation at 4 °C protected from light. Next, 1 mL of flow cytometry buffer was added to each tube, which was then centrifuged at 1200 rpm at room temperature for 5 minutes and supernatant was discarded. This wash step was repeated three times. 250 µL of flow cytometry buffer was added to each flow cytometry tube to re-suspend the cells again, and the cells were mixed thoroughly and detected. Detection results are shown in Table 3 and Figure 3.

**Table 3. Detection results of the binding of hybridoma cell culture supernatants containing anti-PD1 antibodies to PD1-expressing cells**

| Hybridoma cell line | KD-22-0051 84B1-22 | KD-22-0052 89D6-8 | KD-22-0053 92C8-18 | KD-22-0054 107D12-10 | KD-22-0055 113B5-17 | mIgG irrelevant antibody (FGFR2B) |
|---|---|---|---|---|---|---|
| EC50 (µg/mL) | 1.347 | 0.7335 | 1.146 | 0.6855 | 0.7743 | N/A |
| MFI.Max | 65580 | 67125 | 69247 | 67190 | 64579 | 17.6 |

### Example 8 Detection of the blocking of PD1 binding to PDL1-expressing cells by hybridoma cell culture supernatants containing anti-PD1 antibodies

293T cells overexpressing human PDL1 (referred to as "293T-PDL1 cells"; Kyinno, Cat No.: KC-0205) were collected, washed once with FACS buffer, and plated at 2-5×10⁵ cells/well in 96-deep well plates. 50 µL of hybridoma cell culture supernatant and 500 ng of human PD1-hFc (NCBI Reference Sequence: NP_005009.2) were added per well as a premix into the plates, followed by 2 hours of incubation. Next, 400 µL of FACS buffer was added per well to wash twice, and then PE-conjugated anti-human IgG Fc secondary antibody (SouthernBiotech, Cat No.: 2010-09) was added, followed by 1 hour of incubation. 400 µL of FACS buffer was added per well to wash twice again, then the cells were detected. Detection results are shown in Table 4 and Figure 4.

**Table 4. Detection results of the blocking of PD1 binding to PDL1-expressing cells by hybridoma cell culture supernatants containing anti-PD1 antibodies**

| Hybridoma cell line | KD-22-0051 84B1-22 | KD-22-0052 89D6-8 | KD-22-0053 92C8-18 | KD-22-0054 107D12-10 | KD-22-0055 113B5-17 | mIgG irrelevant antibody (FGFR2B) |
|---|---|---|---|---|---|---|
| IC50 (µg/mL) | 3.492 | 2.156 | 2.775 | 2.258 | 2.379 | N/A |

A murine antibody was obtained from the hybridoma cell line KD-22-0054 107D12-10 and was named "107D12-10". Its variable region sequences are as follows (heavy and light chain CDRs are underlined):
> Heavy chain variable region of anti-PD1 murine antibody 107D12-10 (SEQ ID NO. 12; HCDRs: SEQ ID NOs. 28/29/30): 
QVQLKESGGGLVKPGGSLKLSCAASGFTFSDYGMHWVRQAPEKGLEWVAYISSGSYTIYYADTV
RDRFTISRDSAKNTLFLQMTSLRSEDTAMYYCVRRGPGYFGSTALDYWGQGTTLTVSS
> Light chain variable region of anti-PD1 murine antibody 107D12-10 (SEQ ID NO. 13; LCDRs: SEQ ID NOs. 31/33/32):

QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIK

### Example 9 Construction of anti-PD1 humanized antibodies and chimeric antibodies

The heavy and light chain variable regions of the anti-PD1 murine antibody 107D12-10 were humanized to obtain humanized antibody sequences. Coding sequences of the humanized antibody sequences were linked to the coding sequences of the human IgG4 heavy chain constant region (SEQ ID NO. 17) and human kappa light chain constant region (SEQ ID NO. 18), respectively. The obtained coding genes were cloned into eukaryotic expression vectors, expressed, and an anti-PD1 humanized antibody was obtained and named "KA-1807 H107D12-10V4-hIgG4M". Its variable region sequences (SEQ ID NO. 14; and SEQ ID NO. 15) and full-length light chain (SEQ ID NO. 37) are shown below (heavy and light chain CDRs are underlined).

Additionally, two chimeric antibodies were constructed based on the murine antibody 107D12-10.

One chimeric antibody was constructed as follows: coding sequence of the heavy chain variable region of murine antibody 107D12-10 (SEQ ID NO. 12) was linked to the coding sequence of the human IgG4 heavy chain constant region, and coding sequence of the light chain variable region of murine antibody 107D12-10 (SEQ ID NO. 13) was linked to the coding sequence of the human kappa light chain constant region. The obtained coding genes were cloned into eukaryotic expression vectors, expressed, and an anti-PD-1 chimeric antibody was obtained and named "KA-1773 M107D12-10CLC1-hIgG4M". Its variable region sequences (SEQ ID NO. 12; and SEQ ID NO. 13) and full-length light chain sequence (SEQ ID NO. 36) are shown below. Another chimeric antibody was constructed as follows: coding sequence of the heavy chain variable region of murine antibody 107D12-10 (SEQ ID NO. 12) was linked to the coding sequence of the human IgG4 heavy chain constant region, and coding sequence of the light chain variable region shown in SEQ ID NO. 2 (KV4-59-1) was linked to the coding sequence of the human kappa light chain constant region. The obtained coding genes were cloned into eukaryotic expression vectors, expressed, and an anti-PD-1 chimeric antibody was obtained and named "KA-1774 M107D12-10CLC2-hIgG4M". Its variable region sequences (SEQ ID NO. 12; and SEQ ID NO. 2) and full-length light chain sequence (SEQ ID NO. 35) are shown below.
> Heavy chain variable region of anti-PD1 humanized antibody KA-1807 H107D12-10V4-hIgG4M (SEQ ID NO. 14; HCDRs: SEQ ID NOs. 28/29/30): 
QVQLVESGGGLVKPGGSLRLSCAASGFTFSDYGMHWIRQAPGKGLEWVAYISSGSYTIYYADTVR
DRFTISRDSAKNSLYLQMNSLRAEDTAVYYCVRRGPGYFGSTALDYWGQGTTVTVSS
> Light chain variable region of anti-PD1 humanized antibody KA-1807 H107D12-10V4-hIgG4M (SEQ ID NO. 15; LCDRs: SEQ ID NOs. 31/33/32): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIK
> Heavy chain variable region of anti-PD1 murine antibody 107D12-10 (SEQ ID NO. 12; HCDRs: SEQ ID NOs. 28/29/30): 
QVQLKESGGGLVKPGGSLKLSCAASGFTFSDYGMHWVRQAPEKGLEWVAYISSGSYTIYYADTV
RDRFTISRDSAKNTLFLQMTSLRSEDTAMYYCVRRGPGYFGSTALDYWGQGTTLTVSS
> Light chain variable region of anti-PD1 murine antibody 107D12-10 (SEQ ID NO. 13; LCDRs: SEQ ID NOs. 31/33/32): 
QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIK
> KV4-59-1 (SEQ ID NO. 2; LCDRs: SEQ ID NOs. 25/26/32): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIK
> Human heavy chain CH1 and Fc sequence (SEQ ID NO. 17):
   hIgG4-S228P 
ASTKGPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTLMISR
TPEVTCVVVDVSQEDPEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSLSLSLGK
> Human light chain CL sequence (SEQ ID NO. 18):
   hkappa
> Light chain (SEQ ID NO. 35): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTH
QGLSSPVTKSFNRGEC
> Light chain (SEQ ID NO. 36): 
QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTH
QGLSSPVTKSFNRGEC
> Light chain (SEQ ID NO. 37):

DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC

### Example 10 Detection of the binding of anti-PD1 humanized antibodies and chimeric antibodies to PD1-expressing cells

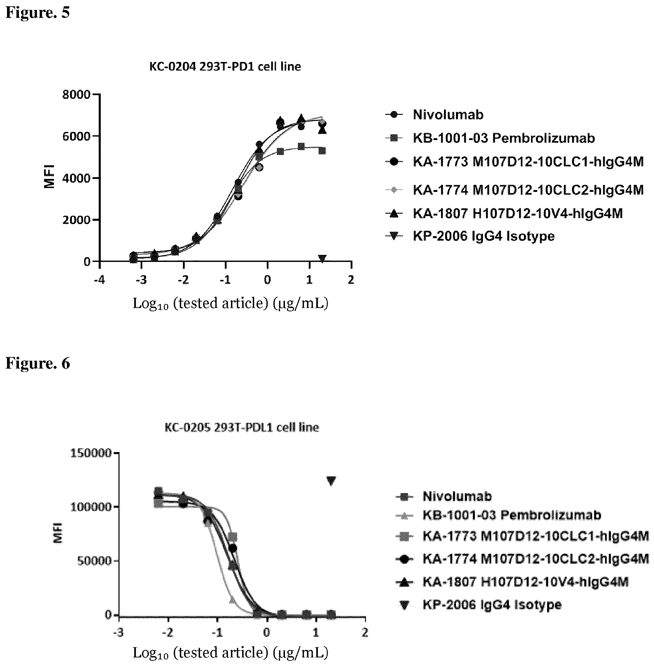

Flow cytometry (FACS) was used to detect the binding of the humanized antibodies and chimeric antibodies to PD1-expressing cells. The experimental procedure followed that described in Example 7, differing in the use of Nivolumab and Pembrolizumab as positive control antibodies, and the addition of Goat anti-human IgG-PE secondary antibody (SouthernBiotech, Cat No.: 2010-09). Detection results are shown in Table 5 and Figure 5.

**Table 5. Detection results of the binding of anti-PD1 humanized antibodies and chimeric antibodies to PD1-expressing cells**

| Antibody | Nivolumab | Pembrolizumab | KA-1773 M107D12-10CLC1-hIgG4M | KA-1774 M107D12-10CLC2-hIgG4M | KA-1807 H107D12-10V4-hIgG4M | KP-2006 IgG4 irrelevant antibody |
|---|---|---|---|---|---|---|
| EC50 (µg/mL) | 0.1450 | 0.1146 | 0.2648 | 0.2723 | 0.1932 | N/A |

### Example 11 Detection of the blocking of PD1 binding to PDL1-expressing cells by anti-PD1 humanized antibody and chimeric antibodies

293T-PDL1 cells were collected, washed once with FACS buffer, and plated at 2-5×10⁵ cells/well in 96-deep well plates. 50 µL of one of the antibodies to be detected (anti-PD1 chimeric antibodies KA-1773 M107D12-10CLC1-hIgG4M and KA-1774 M107D12-10CLC2-hIgG4M and humanized antibody KA-1807 H107D12-10V4-hIgG4M, and control antibodies Nivolumab and Pembrolizumab) and 30 ng of the fusion protein PD1-mFc were added per well as a premix into the plates, followed by 2 hours of incubation. Next, 400 µL of FACS buffer was added per well to wash twice, and then PE-conjugated anti-mouse IgG Fc secondary antibody was added, followed by 1 hour of incubation. 400 µL of FACS buffer was added per well to wash twice again, then the cells were detected. Detection results are shown in Table 6 and Figure 6.

**Table 6. Detection results of the blocking of PD1 binding to PDL1-expressing cells by anti-PD1 antibodies**

| Antibody | Nivolumab | KB-1001-03 Pembrolizumab | KA-1773 M107D12-10CLC1-hIgG4M | KA-1774 M107D12-10CLC2-hIgG4M | KA-1807 H107D12-10V4-hIgG4M | IgG4 irrelevant antibody |
|---|---|---|---|---|---|---|
| IC50 (µg/mL) | 0.1630 | 0.09672 | 0.2557 | 0.2179 | 0.1578 | N/A |

### Example 12 Construction of anti-LAG3 humanized antibodies and chimeric antibodies

The heavy chain variable region of the anti-LAG3 murine antibody 28G2 was humanized to obtain a humanized antibody sequence (SEQ ID NO. 19); additionally, the light chain variable region of the anti-PD1 humanized antibody provided in Example 9 (SEQ ID NO. 15) was used as the light chain variable region to construct an anti-LAG3 humanized antibody. Coding sequences of the humanized antibody sequences were linked to the coding sequences of the human IgG4 heavy chain constant region (SEQ ID NO. 17) and human kappa light chain constant region (SEQ ID NO. 18), respectively. The obtained coding genes were cloned into eukaryotic expression vectors, expressed, and anti-LAG3 humanized antibodies were obtained and named "KA-1780 H28G2-7V2-hIgG4M" and "KA-1782 H28G2-7V4-hIgG4M". Antibody KA-1782 H28G2-7V4-hIgG4M's variable region sequences (SEQ ID NO. 19; and SEQ ID NO. 15) and full-length light chain (SEQ ID NO. 37) are shown below.

Additionally, two chimeric antibodies were constructed based on the murine antibody 28G2 and the murine antibody 107D12-10. One chimeric antibody was constructed as follows: coding sequence of the heavy chain variable region of murine antibody 28G2 (SEQ ID NO. 10) was linked to the coding sequence of the human IgG4 heavy chain constant region, and coding sequence of the light chain variable region of murine antibody 107D12-10 (SEQ ID NO. 13) was linked to the coding sequence of the human kappa light chain constant region. The obtained coding genes were cloned into eukaryotic expression vectors, expressed, and an anti-LAG3 chimeric antibody was obtained and named "KA-1728 M28G2-7CLC1-hIgG4M". Its variable region sequences (SEQ ID NO. 10; and SEQ ID NO. 13) and full-length light chain sequence (SEQ ID NO. 36) are shown below. Another chimeric antibody was constructed as follows: coding sequence of the heavy chain variable region of murine antibody 28G2 (SEQ ID NO. 10) was linked to the coding sequence of the human IgG4 heavy chain constant region, and coding sequence of the light chain variable region shown in SEQ ID NO. 2 (KV4-59-1) was linked to the coding sequence of the human kappa light chain constant region. The obtained coding genes were cloned into eukaryotic expression vectors, expressed, and an anti-LAG3 chimeric antibody was obtained and named "KA-1733 M28G2-7CLC2-hIgG4M". Its variable region sequences (SEQ ID NO. 10; and SEQ ID NO. 2) and full-length light chain sequence (SEQ ID NO. 35) are shown below.
> Heavy chain variable region of anti-LAG3 humanized antibody KA-1782 H28G2-7V4-hIgG4M (SEQ ID NO. 19; HCDRs: SEQ ID NOs. 22/34/24): 
QVQLQESGPGLVKPSETLSLTCTVSGYSFTSGYYWNWIRQPPGKGLEWIGYVSYFGTANYNPALK
NRVTISRDTSKNQFSLKLSSVTAADTAVYYCARGEEWDWSFDVWGQGTTVTVSS
> Light chain variable region of anti-LAG3 humanized antibody KA-1782 H28G2-7V4-hIgG4M (light chain variable region of anti-PD1 humanized antibody KA-1807 H107D12-10V4-hIgG4M; SEQ ID NO. 15; LCDRs: SEQ ID NOs. 31/33/32): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIK
> Heavy chain variable region of anti-LAG3 murine antibody 28G2 (SEQ ID NO. 10; HCDRs: SEQ ID NOs. 22/23/24): 
DVKLQESGPGLVKPSQSLSLTCSVTGYSFTSGYYWNWIRLFPGNKLEWMGYVSYFGTNNYNPSL
KNRFSITRDTSKNQFFLKLNSVSTEDTATYYCARGEEWDWSFDVWGTGTTVTVSS
> Light chain variable region of anti-PD1 murine antibody 107D12-10 (SEQ ID NO. 13; LCDRs: SEQ ID NOs. 31/33/32): 
QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIK
> KV4-59-1 (SEQ ID NO. 2; LCDRs: SEQ ID NOs. 25/26/32): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIK
> Human heavy chain CH1 and Fc sequence (SEQ ID NO. 17):
   hIgG4-S228P 
ASTKGPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTLMISR
TPEVTCVVVDVSQEDPEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCLVKGFYPSDIAVEWESNG
QPENNYKTTPPVLDSDGSFFLYSRLTVDKSRWQEGNVFSCSVMHEALHNHYTQKSLSLSLGK
> Human light chain CL sequence (SEQ ID NO. 18):
   hkappa
> Light chain (SEQ ID NO. 35): 
QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTH
QGLSSPVTKSFNRGEC
> Light chain (SEQ ID NO. 36): 
QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTH
QGLSSPVTKSFNRGEC
> Light chain (SEQ ID NO. 37):

DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIKRTVAAPSVFIFPPSDEQLKSGTASVVCL

LNNFYPREAKVQWKVDNALQSGNSQESVTEQDSKDSTYSLSSTLTLSKADYEKHKVYACEVTHQ
GLSSPVTKSFNRGEC

### Example 13 Detection of the binding of anti-LAG3 humanized antibodies and chimeric antibodies to LAG3-expressing cells

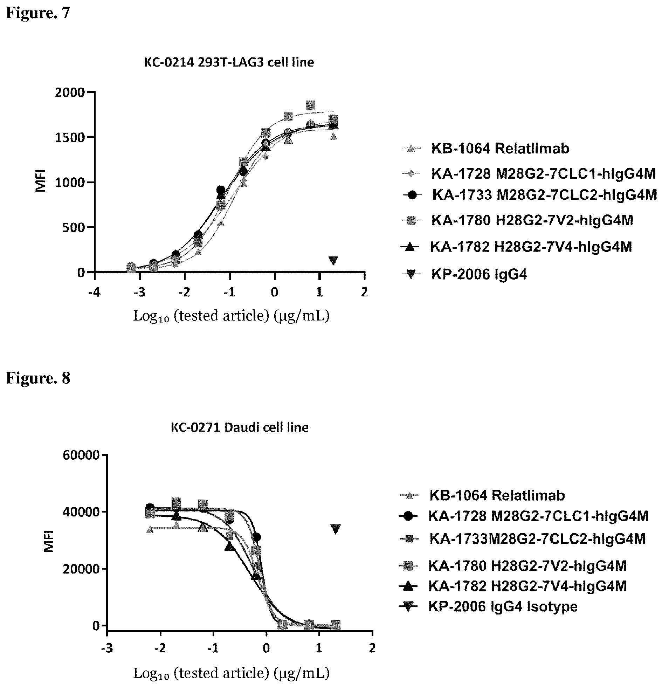

Flow cytometry (FACS) was used to detect the binding of the humanized antibodies and chimeric antibodies provided in Example 12 to LAG3-expressing cells. The experimental procedure followed that described in Example 4, differing in the use of Relatlimab as a positive control antibody and the addition of Goat anti-human IgG-PE secondary antibody (SouthernBiotech, Cat No.: 2010-09). Detection results are shown in Table 7 and Figure 7.

**Table 7. Detection results of the binding of anti-LAG3 humanized antibodies and chimeric antibodies to LAG3-expressing cells**

| Antibody | KB-1064 Relatlimab | KA-1728 M28G2-7CLC1-hIgG4M | KA-1733 M28G2-7CLC2-hIgG4M | KA-1780 H28G2-7V2-hIgG4M | KA-1782 H28G2-7V4-hIgG4M | KP-2006 IgG4 irrelevant antibody |
|---|---|---|---|---|---|---|
| EC50 (µg/mL) | 0.1206 | 0.1185 | 0.06492 | 0.09406 | 0.06741 | N/A |

### Example 14 Detection of the blocking of LAG3 binding to tumor cells by anti-LAG3 humanized antibodies and chimeric antibodies

Daudi cells were collected, washed once with FACS buffer, and plated at 2-5×10⁵ cells/well in 96-deep well plates. 50 µL of one of the antibodies to be detected (chimeric antibodies KA-1728 M28G2-7CLC1-hIgG4M and KA-1733 M28G2-7CLC2-hIgG4M, humanized antibodies KA-1780 H28G2-7V2-hIgG4M and KA-1782 H28G2-7V4-hIgG4M, and control antibody Relatlimab) and 500 ng of human LAG3-mFc were added per well as a premix into the plates, followed by 2 hours of incubation. Next, 400 µL of FACS buffer was added per well to wash twice, and then the PE-conjugated anti-mouse IgG Fc secondary antibody was added, followed by 1 hour of incubation. 400 µL of FACS buffer was added per well to wash twice again, then the cells were detected. Detection results are shown in Table 8 and Figure 8.

**Table 8. Detection results of the blocking of LAG3 binding to tumor cells by anti-LAG3 humanized antibodies and chimeric antibodies**

| Antibody | KB-1064 Relatlimab | KA-1728 M28G2-7CLC1-hIgG4M | KA-1733 M28G2-7CLC2-hIgG4M | KA-1780 H28G2-7V2-hIgG4M | KA-1782 H28G2-7V4-hIgG4M | KP-2006 IgG4 irrelevant antibody |
|---|---|---|---|---|---|---|
| IC50 (µg/mL) | 0.6892 | 0.8047 | 0.5632 | 0.7418 | 0.4762 | N/A |

### Example 15 Construction of PD1 ×LAG3 bispecific antibodies

Coding sequence of the amino acid sequence shown in SEQ ID NO. 15 or SEQ ID NO. 16 (see below, a sequence obtained by humanizing KV4-59-1) was linked to the coding sequence of the human kappa light chain constant region (SEQ ID NO. 18), thereby obtaining common light chain 1 (SEQ ID NO. 37) or common light chain 2 (SEQ ID NO. 38) which would be used for constructing a bispecific antibody.

Coding sequence of the heavy chain variable region of the anti-PD1 humanized antibody was linked to the coding sequence of the following human heavy chain CH1 and Fc sequence ("Knob") and coding sequence of the heavy chain variable region of the anti-LAG3 humanized antibody was linked to the coding sequence of the following human heavy chain CH1 and Fc sequence ("Hole"). Alternatively, coding sequence of the heavy chain variable region of the anti-LAG3 humanized antibody was linked to the coding sequence of the following human heavy chain CH1 and Fc sequence ("Knob") and coding sequence of the heavy chain variable region of the anti-PD1 humanized antibody was linked to the coding sequence of the following human heavy chain CH1 and Fc sequence ("Hole").

The obtained coding genes were cloned into eukaryotic expression vectors, thereby obtaining three recombinant expression plasmids (encoding the common light chain 1 or common light chain 2, encoding a PD1-targeting heavy chain, and encoding a LAG3-targeting heavy chain). HEK293F expression system was used for transient co-transfection of three plasmids to produce PD1×LAG3 bispecific antibodies. Briefly, for a 1 L shaker flask, HEK293F cells were seeded at 1×10⁶ cells/mL in 250 mL of culture medium, and incubated at 110 rpm, 5% CO₂; next day, the pre-prepared three expression plasmids were mixed with transfection reagent at a certain ratio, and the transfection complex was added to the cells at a cell density of 2×10⁶ cells/mL; 24 hours later, nutrients and DNA inhibitors were supplemented; after 5-7 days of cell culture, expression supernatants were collected, centrifuged, filtered, and purified using a MabSelectSure affinity chromatography column (GE Healthcare). The purified antibody was detected for purity by SDS-PAGE electrophoresis, and antibody concentration was detected using Nanodrop.

The domains comprised in the obtained PD1×LAG3 bispecific antibodies are shown in Table 9.

**Table 9. Bispecific antibodies comprising common light chain**

| Bispecific antibody | PD 1-targeting heavy chain | | LAG3-targeting heavy chain | | Common light chain | | |
|---|---|---|---|---|---|---|---|
| | Heavy chain variable region | Heavy chain constant region | Heavy chain variable region | Heavy chain constant region | Light chain variable region | Light chain constant region | Light chain |
| KA-1835 H28G27V4×H107D1210 -hIgG4M | SEQ ID NO. 14 | SEQ ID NO. 21 [Hole] | SEQ ID NO. 19 | SEQ ID NO. 20 [Knob] | SEQ ID NO. 15 | SEQ ID NO. 18 | SEQ ID NO. 37 |
| KA-1836 H107D1210×H28G27V4-hIgG4M | SEQ ID NO. 14 | SEQ ID NO. 20 [Knob] | SEQ ID NO. 19 | SEQ ID NO. 21 [Hole] | SEQ ID NO. 15 | SEQ ID NO. 18 | SEQ ID NO. 37 |
| KA-1793 H28G27V4×H107D1210-hIgG4M | SEQ ID NO. 14 | SEQ ID NO. 21 [Hole] | SEQ ID NO. 19 | SEQ ID NO. 20 [Knob] | SEQ ID NO. 16 | SEQ ID NO. 18 | SEQ ID NO. 38 |

> Heavy chain variable region of anti-PD1 humanized antibody KA-1807 H107D12-10V4-hIgG4M (SEQ ID NO. 14; HCDRs: SEQ ID NOs. 28/29/30): 
QVQLVESGGGLVKPGGSLRLSCAASGFTFSDYGMHWIRQAPGKGLEWVAYISSGSYTIYYADTVR
DRFTISRDSAKNSLYLQMNSLRAEDTAVYYCVRRGPGYFGSTALDYWGQGTTVTVSS
> Heavy chain variable region of anti-LAG3 humanized antibody KA-1782 H28G2-7V4-hIgG4M (SEQ ID NO. 19; HCDRs: SEQ ID NOs. 22/34/24): 
QVQLQESGPGLVKPSETLSLTCTVSGYSFTSGYYWNWIRQPPGKGLEWIGYVSYFGTANYNPALK
NRVTISRDTSKNQFSLKLSSVTAADTAVYYCARGEEWDWSFDVWGQGTTVTVSS
> Light chain variable region of anti-PD1 humanized antibody KA-1807 H107D12-10V4-hIgG4M (SEQ ID NO. 15; LCDRs: SEQ ID NOs. 31/33/32): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIK
> Humanized sequence of KV4-59-1 (SEQ ID NO. 16; LCDRs: SEQ ID NOs. 25/26/32): 
DIQITQSPSSLSASVGDRVTITCSASSSVSYMHWYQQKPGKAPKRLIYDTSKLASGVPSRFSGSGSG
TDYTLTISSLQPEDFATYYCQQWSSNPPYTFGQGTRLEIK
> Human heavy chain CH1 and Fc sequence ("Knob"; hIgG4; SEQ ID NO. 20): 
ASTKGPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTLMISR
TPEVTCVVVDVSQEDPEVQFNWYVDGVEVHNAKTKPREEQFNSTYRVVSVLTVLHQDWLNGKE
YKCKVSNKGLPSSIEKTISKAKGQPREPQVYTLPPCQEEMTKNQVSLWCLVKGFYPSDIAVEWESN
GQPENNYKTTPPVLDSDGSFFLYSRLTVDKSRWQEGNVFSCSVMHEALHNHYTQKSLSLSLG
> Human heavy chain CH1 and Fc sequence ("Hole"; hIgG4; SEQ ID NO. 21): 
ASTKGPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTLMISR
TPEVTCVVVDVSQEDPEVQFNWYVDGVEVHNAKTKPREEQFNSTYRVVSVLTVLHQDWLNGKE
YKCKVSNKGLPSSIEKTISKAKGQPREPQVCTLPPSQEEMTKNQVSLSCAVKGFYPSDIAVEWESNG
QPENNYKTTPPVLDSDGSFFLVSRLTVDKSRWQEGNVFSCSVMHEALHNHYTQKSLSLSLG
> Human light chain CL sequence (SEQ ID NO. 18):
   hkappa
> Light chain (SEQ ID NO. 37): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC
> Light chain (SEQ ID NO. 38):

DIQITQSPSSLSASVGDRVTITCSASSSVSYMHWYQQKPGKAPKRLIYDTSKLASGVPSRFSGSGSG
TDYTLTISSLQPEDFATYYCQQWSSNPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC

### Example 16 Detection of the binding of PD1 ×LAG3 bispecific antibodies having common light chain 1 to PD1-expressing cells and LAG3-expressing cells

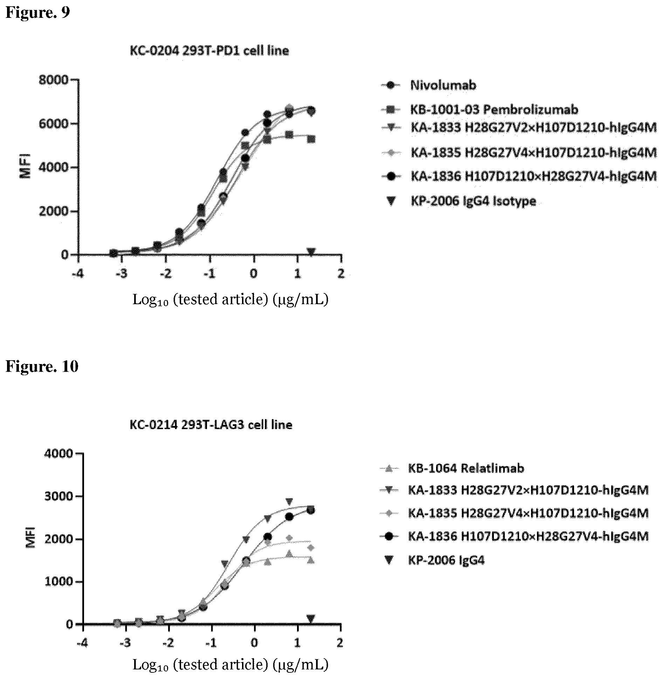

Using the 293T-PD1 cells and the 293T-LAG3 cells respectively, flow cytometry (FACS) was used to detect the binding of the PD1×LAG3 bispecific antibodies to PD1-expressing cells (293T-PD1 cells) and LAG3-expressing cells (293T-LAG3 cells). The experimental procedures followed those described in Example 4 and Example 7 respectively. Detection results are shown in Table 10 and Figure 9 (target protein: PD1), and Table 11 and Figure 10 (target protein: LAG3).

**Table 10. Detection results of the binding of PD1 ×LAG3 bispecific antibodies to PD1-expressing cells**

| Antibody | Nivolumab | KB-1001-03 Pembrolizumab | KA-1833 H28G27V2×H107D1 210-hIgG4M | KA-1835 H28G27V4×H107D12 10-hIgG4M | KA-1836 H107D1210×H28G27V 4-hIgG4M |
|---|---|---|---|---|---|
| EC50 (µg/mL) | 0.1450 | 0.1146 | 0.4102 | 0.6512 | 0.3297 |

**Table 11. Detection results of the binding of PD1 ×LAG3 bispecific antibodies to LAG3-expressing cells**

| Antibody | KB-1064 Relatlimab | KA-1833 H28G27V2×H107D1210-hIgG4M | KA-1835 H28G27V4×H107D1210-hIgG4M | KA-1836 H107D1210×H28G27V4-hIgG4M |
|---|---|---|---|---|
| EC50 (µg/mL) | 0.1206 | 0.2498 | 0.2276 | 0.5575 |

### Example 17 Detection of the blocking of PD1 binding to PDL1-expressing cells and LAG3 binding to tumor cells by PD1 ×LAG3 bispecific antibodies having common light chain 1

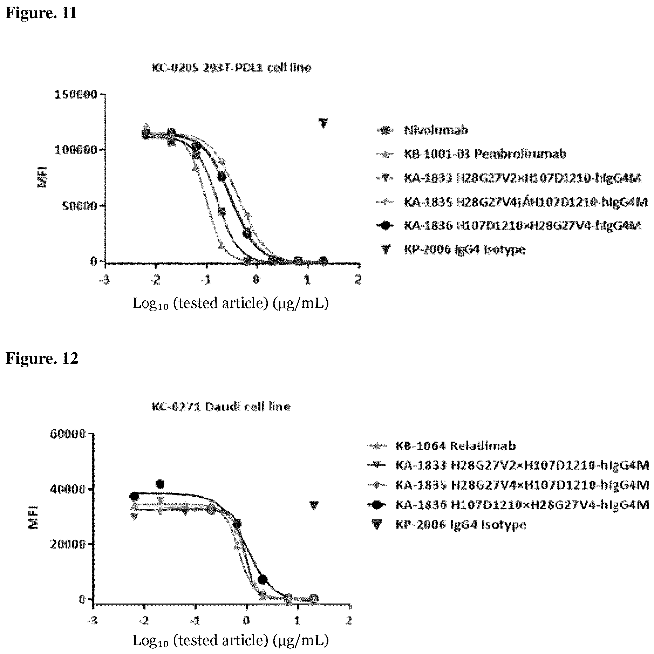

Using the 293T-PDL1 cells and Daudi cells respectively, flow cytometry (FACS) was used to detect the blocking of PD1 binding to PDL1-expressing cells and LAG3 binding to tumor cells by PD1×LAG3 bispecific antibodies. The experimental procedures followed those described in Example 5 and Example 8 respectively. Detection results are shown in Table 12 and Figure 11 (target protein: PD1), and Table 13 and Figure 12 (target protein: LAG3).

**Table 12. Detection results of the blocking of PD1 binding to PDL1-expressing cells by PD1 ×LAG3 bispecific antibodies**

| Antibod y | Nivoluma b | KB-1001-03 Pembrolizuma b | KA-1833 H28G27V2×H107D121 0-hIgG4M | KA-1835 H28G27V4×H107D121 0-hIgG4M | KA-1836 H107D1210×H28G27V 4-hIgG4M |
|---|---|---|---|---|---|
| IC50 (µg/mL) | 0.1630 | 0.09672 | 0.3135 | 0.4355 | 0.3024 |

**Table 13. Detection results of the blocking of LAG3 binding to tumor cells by PD1×LAG3 bispecific antibodies**

| Antibody | KB-1064 Relatlimab | KA-1833 H28G27V2×H107D1210-hIgG4M | KA-1835 H28G27V4×H107D1210-hIgG4M | KA-1836 H107D1210×H28G27V4-hIgG4M |
|---|---|---|---|---|
| IC50 (µg/mL) | 0.6892 | 0.9227 | 0.8793 | 0.9720 |

### Example 18 Detection of affinity of PD1×LAG3 bispecific antibodies having common light chain 1 for PD1 and LAG3

ForteBio Octet instrument was used to detect the affinity of PD1 ×LAG3 bispecific antibodies for PD1 and LAG3. The mobile phases were PD1-His (Kyinno, Cat No.: KP-1023) which was diluted to concentrations of 400 nM, 100 nM, 50 nM, 25 nM, 12.5 nM, 6.25 nM, and 3.12 nM and LAG3-His (ACRO, Cat No.: LA3-H5222) which was diluted to concentrations of 200 nM, 100 nM, 50 nM, 25 nM, 12.5 nM, 6.25 nM, and 3.12 nM, detected simultaneously. Results are shown in Table 13 and Table 14.

**Table 13. Affinity of PD1 ×LAG3 bispecific antibodies for PD1**

| Antibody | KD (M) | kon(1/Ms) | kdis(1/s) | RMax | FullR² |
|---|---|---|---|---|---|
| KA-1833 H28G27V2×H107D1210-hIgG4M | 3.15E-09 | 1.95E+05 | 6.13E-04 | 0.0775 | 0.9743 |
| KA-1835 H28G27V4×H107D1210-hIgG4M | 4.73E-09 | 2.22E+05 | 1.05E-03 | 0.1264 | 0.969 |
| KA-1836 H107D1210×H28G27V4-hIgG4M | 6.55E-09 | 1.78E+05 | 1.16E-03 | 0.1029 | 0.9954 |
| KA-1807 H107D12-10V4-hlgG4M | 6.96E-09 | 2.05E+05 | 1.43E-03 | 0.2883 | 0.9916 |
| Pembrolizumab | 3.15E-09 | 1.16E+06 | 3.65E-03 | 0.2666 | 0.9848 |

**Table 14. Affinity of PD1×LAG3 bispecific antibodies for LAG3**

| Antibody | KD (M) | kon(1/Ms) | kdis(1/s) | RMax | 2 FullR |
|---|---|---|---|---|---|
| KA-1833 H28G27V2×H107D1210-hIgG4M | 6.01E-11 | 1.13E+06 | 6.80E-05 | 0.6131 | 0.9964 |
| KA-1835 H28G27V4×H107D1210-hIgG4M | 1.68E-10 | 1.16E+06 | 1.95E-04 | 0.5902 | 0.996 |
| KA-1836 H107D1210×H28G27V4-hIgG4M | 1.80E-10 | 1.48E+06 | 2.66E-04 | 0.4466 | 0.9943 |
| KA-1807 H28G2-7V2-hIgG4M | 6.68E-11 | 8.73E+05 | 5.83E-05 | 0.8539 | 0.9973 |
| KA-1782 H28G2-7V4-hIgG4M | 1.74E-10 | 8.81E+05 | 1.53E-04 | 0.6632 | 0.9972 |
| Relatlimab | 3.03E-10 | 7.33E+05 | 2.23E-04 | 0.8524 | 0.9971 |

### Example 19 Detection of the binding of PD1 ×LAG3 bispecific antibodies having common light chain 2 to PD1-expressing cells and LAG3-expressing cells

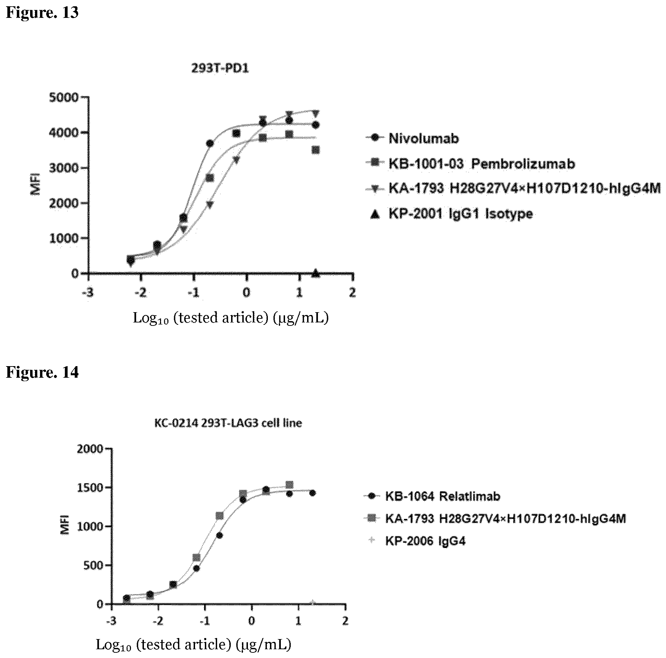

Using the 293T-PD1 cells and the 293T-LAG3 cells respectively, flow cytometry (FACS) was used to detect the binding of the PD1×LAG3 bispecific antibodies to PD1-expressing cells (293T-PD1 cells) and LAG3-expressing cells (293T-LAG3 cells). The experimental procedures followed those described in Example 4 and Example 7 respectively. Detection results are shown in Table 16 and Figure 13 (target protein: PD1), and Table 17 and Figure 14 (target protein: LAG3).

**Table 16. Detection results of the binding of PD1 ×LAG3 bispecific antibodies to PD1-expressing cells**

| Antibody | Nivolumab | KB-1001-03 Pembrolizumab | KA-1793 H28G27V4×H107D1210-hIgG4M |
|---|---|---|---|
| EC50 (µg/mL) | 0.09230 | 0.1085 | 0.3004 |
| MFI.Max | 4350 | 3957 | 4524 |

**Table 17. Detection results of the binding of PD1 ×LAG3 bispecific antibodies to LAG3-expressing cells**

| Antibody | KB-1064 Relatlimab | KA-1793 H28G27V4×H107D1210-hIgG4M |
|---|---|---|
| EC50 (µg/mL) | 0.1502 | 0.09312 |
| MFI.Max | 1480 | 1539 |

### Example 20 Detection of the blocking of PD1 binding to PDL1-expressing cells and LAG3 binding to tumor cells by PD1 ×LAG3 bispecific antibodies having common light chain 2

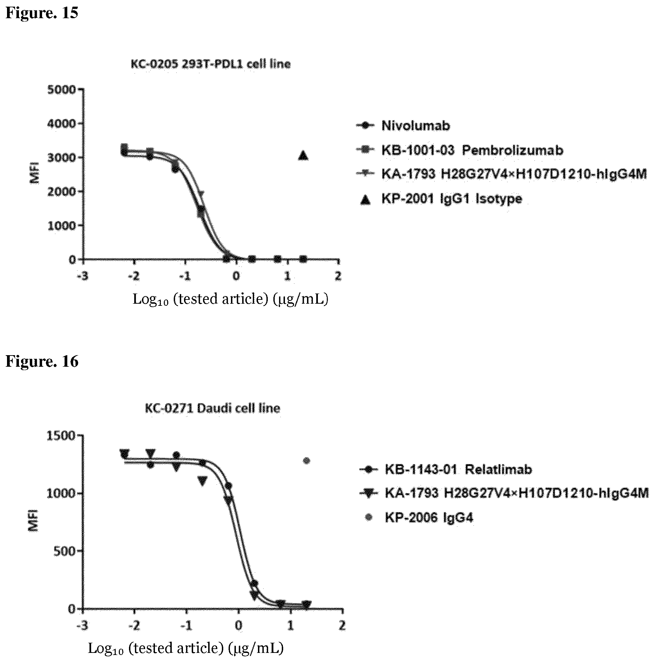

Using the 293T-PDL1 cells and Daudi cells respectively, flow cytometry (FACS) was used to detect the blocking of PD1 binding to PDL1-expressing cells and LAG3 binding to tumor cells by PD1 ×LAG3 bispecific antibodies. The experimental procedures followed those described in Example 5 and Example 8 respectively. Detection results are shown in Table 18 and Figure 15 (target protein: PD1), and Table 19 and Figure 16 (target protein: LAG3).

**Table 18. Detection results of the blocking of PD1 binding to PDL1-expressing cells by PD1 ×LAG3 bispecific antibodies**

| Antibody | Nivolumab | KB-1001-03 Pembrolizumab | KA-1793 H28G27V4xH107D1210-hIgG4M | KP-2001 IgG1 irrelevant antibody |
|---|---|---|---|---|
| IC50 (µg/mL) | 0.1892 | 0.1659 | 0.2317 | N/A |

**Table 19. Detection results of the blocking of LAG3 binding to tumor cells by PD1×LAG3 bispecific antibodies**

| Antibody | KB-1143-01 Relatlimab | KA-1793 H28G27V4×H107D1210-hIgG4M | KP-2006 IgG4 irrelevant antibody |
|---|---|---|---|
| IC50 (µg/mL) | 1.068 | 0.88 | N/A |

### Example 21 Detection of affinity of PD1×LAG3 bispecific antibodies having common light chain 2 for PD1 and LAG3

ForteBio Octet instrument was used to detect the affinity of PD1×LAG3 bispecific antibodies for PD1 and LAG3. The mobile phases were PD1-His (Kyinno, Cat No.: KP-1023) which was diluted to concentrations of 400 nM, 100 nM, 50 nM, 25 nM, 12.5 nM, 6.25 nM, and 3.12 nM and LAG3-His (ACRO, Cat No.: LA3-H5222) which was diluted to concentrations of 200 nM, 100 nM, 50 nM, 25 nM, 12.5 nM, 6.25 nM, and 3.12 nM, detected simultaneously. Results are shown in Table 20 and Table 21.

**Table 20. Affinity of PD1 ×LAG3 bispecific antibodies for PD1**

| Antibody | KD (M) | kon(1/Ms) | kdis(1/s) | RMax | 2 FullR |
|---|---|---|---|---|---|
| H28G27V4×H107D1210-hIgG4M | 6.87E-09 | 3.72E+05 | 2.55E-03 | 0.1237 | 0.959 |

**Table 21. Affinity of PD1×LAG3 bispecific antibodies for LAG3**

| Antibody | KD (M) | kon(1/Ms) | kdis(1/s) | RMax | 2 FullR |
|---|---|---|---|---|---|
| H28G27V4×H107D1210-hIgG4M | 1.75E-10 | 6.33E+05 | 1.11E-04 | 0.9312 | 0.9989 |

### Example 22 Preparation of hybridoma cells secreting anti-PDL1 antibodies

Mice constructed in Example 2 were immunized with a fusion protein (referred to as "PDL1-mFc"; Kyinno, Cat No.: KP-1008) of the human PDL1 extracellular domain and mouse Fc. The experimental procedure followed that described in Example 3. Mice with higher titers were selected, from which sera were collected. Then the mice were dissected, their spleens were taken and spleen cells were isolated. The spleen cells were fused with cultured myeloma cells, and hybridoma cells were obtained.

ELISA plates were coated with PDL1-his. The binding activity of hybridoma cell culture supernatants to the PDL1-his was detected by ELISA, yielding multiple positive hybridoma cell lines secreting anti-PDL1 antibodies.

### Example 23 Detection of the binding of hybridoma cell culture supernatants containing anti-PDL1 antibodies to PDL1-expressing cells

293T-PDL1 cells overexpressing human PDL1 (Kyinno, Cat No.: KC-0205) were prepared as a cell suspension at a concentration of 1×10⁷ cells/mL in PBS containing 2% FBS.

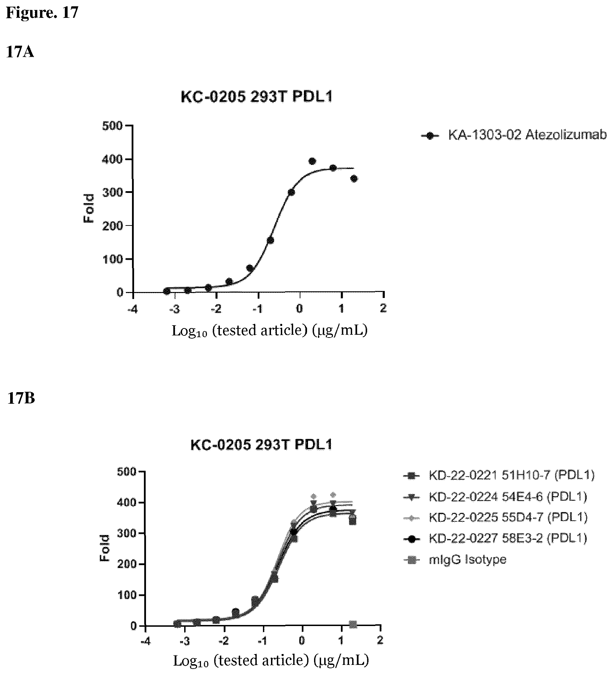

Flow cytometry was used to detect the binding of hybridoma cell culture supernatants containing anti-PDL1 antibodies to 293T-PDL1 cells. The experimental procedure followed that described in Example 4, differing in the use of Atezolizumab (Kyinno, Cat No.: KA-1303-02) as positive control antibody as well as the addition of 5 µL of PE-conjugated anti-mouse IgG Fc secondary antibody to detect the binding of the hybridoma cell culture supernatants and the addition of Goat anti-human IgG-PE secondary antibody (SouthernBiotech, Cat No.: 2010-09) to detect the binding of the positive control antibody Atezolizumab. Detection results are shown in Table 22 and Figure 17.

**Table 22. Detection results of the binding of hybridoma cell culture supernatants containing anti-PDL1 antibodies to PDL1-expressing cells**

| Hybridoma cell line | Atezolizumab | KD-22-0221 51H10-7 | KD-22-0224 54E4-6 | KD-22-0225 55D4-7 | KD-22-0227 58E3-2 | mIgG irrelevant antibody |
|---|---|---|---|---|---|---|
| EC50 (µg/mL) | 0.2443 | 0.2575 | 0.2376 | 0.2301 | 0.2452 | N/A |
| Top | 370.9 | 363 | 390.9 | 402 | 354.5 | N/A |

### Example 24 Detection of the blocking of PDL1-mFc binding to PD1-expressing cells by hybridoma cell culture supernatants containing anti-PDL1 antibodies

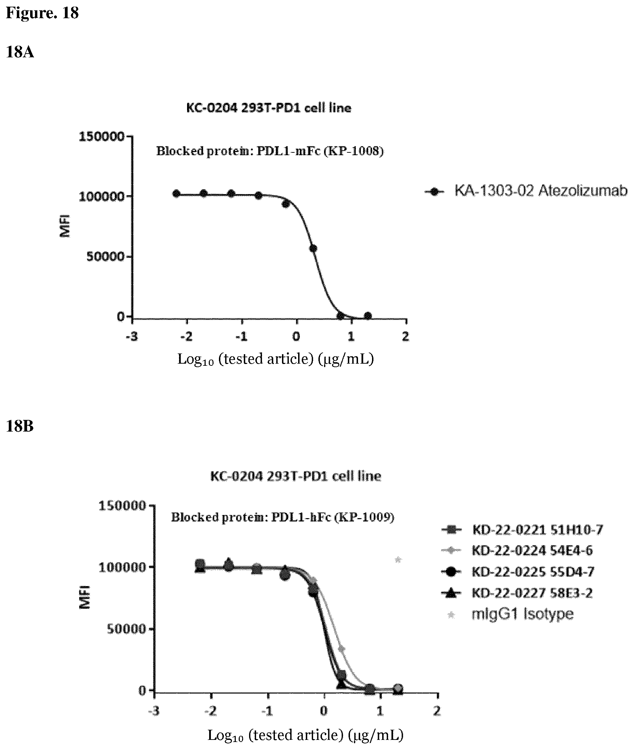

293T cells overexpressing human PD1 (referred to as "293T-PD1 cells"; Kyinno, Cat No.: KC-0204) were washed once with FACS buffer, and plated at 2-5×10⁵ cells/well in 96-deep well plates. 50 µL of hybridoma cell culture supernatant or positive control antibody Atezolizumab and 500 ng of human PDL1-mFc (Kyinno, Cat No.: KP-1008) or PDL1-hFc (Kyinno, Cat No.: KP-1009) (NCBI Reference Sequence: NP_054862.1) were added per well as a premix into the plates, followed by 2 hours of incubation. Next, 400 µL of FACS buffer was added per well to wash twice, and then Goat anti-human IgG-PE secondary antibody (SouthernBiotech, Cat No.: 2010-09) or PE conjugated anti-mouse IgG Fc secondary antibody (Biolegend, Cat No.: 405307) was added, followed by 1 hour of incubation. 400 µL of FACS buffer was added per well to wash twice again, then the cells were detected. Detection results are shown in Table 23 and Figure 18.

**Table 23. Detection results of the blocking of PDL1 binding to PD1-expressing cells by hybridoma cell culture supernatants containing anti-PDL1 antibodies**

| Hybridoma cell line | Atezolizumab | KD-22-0221 51H10-7 | KD-22-0224 54E4-6 | KD-22-0225 55D4-7 | KD-22-0227 58E3-2 | mIgG irrelevant antibody |
|---|---|---|---|---|---|---|
| IC50 (µg/mL) | 2.184 | 1.060 | 1.498 | 1.002 | 0.9857 | N/A |

A murine antibody was obtained from the hybridoma cell line KD-22-0227 58E3-2 and was named "58E3-2". Its variable region sequences are as follows (heavy and light chain CDRs are underlined and identified according to the KABAT numbering scheme):
> Heavy chain variable region of anti-PDL1 murine antibody 58E3-2 (SEQ ID NO. 39; HCDRs: SEQ ID NOs. 48/49/50): 
QVQLKESGGGLVKPGGSLKLSCAASGFTFSDYGIHWVRQAPEKGLEWVAFISSGSSTIYYADTVK
GRFTISRDNAKNTLFLQMTSLRSEDTAMYYCTRRDFYGSADAMDYWGQGTSVTVSS
> Light chain variable region of anti-PDL1 murine antibody 58E3-2 (KV4-59-1; SEQ ID NO. 2; LCDRs: SEQ ID NOs. 25/26/32):

QIVLTQSPAIMSASPGEKVTMTCSASSSVSYMHWYQQKSGTSPKRWIYDTSKLASGVPARFSGSGS
GTSYSLTISSMEAEDAATYYCQQWSSNPPYTFGSGTKLEIK

### Example 25 Detection of the blocking of PDL1/PD1 signaling by anti-PDL1 murine antibodies

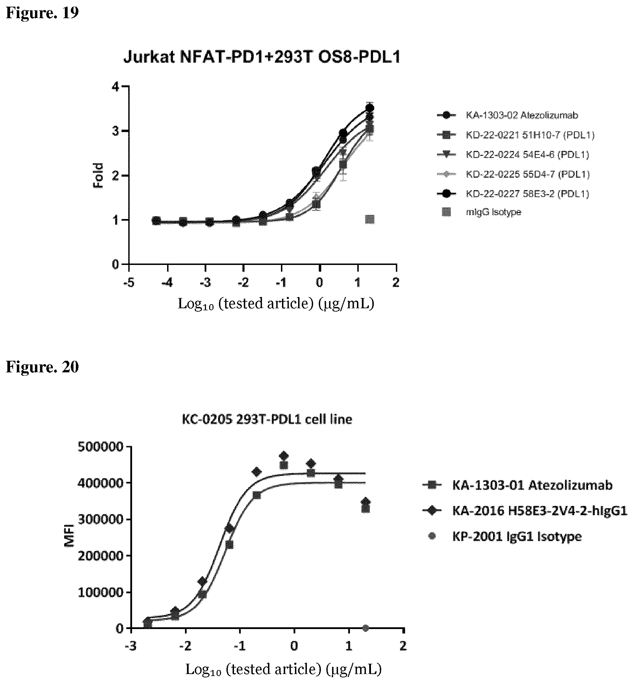

Jurkat NFAT-PD1-luc cells (Kyinno, Cat No.: KC-1503; expressing luciferase upon NFAT signaling activation) and 293T OS8-hPDL1 cells (Kyinno, Cat No.: KC-1148; expressing surface-bound OKT3 antibody) were adjusted to a cell density of 20000 cells/45 µL in RPMI-1640 + 10% FBS medium respectively, and then 45 µL of each of the cell types was added per well into 96-well plates. 10 µL of serially diluted positive control antibody (Atezolizumab) or one of the anti-PDL1 murine antibodies were added to the plates per well, followed by 6 hours of co-culture. Subsequently, 50 µL of Bright-Lite solution (Vazyme, Cat No.: DD1204) was added to each well and luciferase activity was detected. Wells without antibody added served as the background activation control. Fold increase in activation (relative to background) was plotted against antibody concentration, and IC₅₀ values for the anti-PDL1 antibodies were calculated. Detection results are shown in Table 24 and Figure 19.

**Table 24. Detection results of the blocking of PDL1/PD1 signaling by anti-PDL1 murine antibodies**

| Antibody | KA-1303-02 Atezolizumab | KD-22-0221 51H10-7 | KD-22-0224 54E4-6 | KD-22-0225 55D4-7 | KD-22-0227 58E3-2 | mIgG irrelevant antibody |
|---|---|---|---|---|---|---|
| IC50 (µg/mL) | 0.900 | 2.580 | 1.039 | 2.133 | 1.001 | N/A |
| Top | 3.314 | 3.047 | 3.133 | 2.948 | 3.518 | N/A |

### Example 26 Detection of affinity of anti-PDL1 murine antibodies for PDL1

ForteBio Octet instrument was used to detect the affinity of anti-PDL1 murine antibodies for PDL1. The mobile phases were PDL1-His (Kyinno, Cat No.: KP-1022) which was diluted to concentrations of 200 nM, 100 nM, 50 nM, 25 nM, 12.5 nM, 6.25 nM, and 3.12 nM. Results are shown in Table 25.

**Table 25. Affinity of anti-PDL1 murine antibodies for PDL1**

| Antibody | KD (M) | kon(1/Ms) | kdis(1/s) |
|---|---|---|---|
| KD-22-0221 51H10-7 | 3.97E-09 | 4.87E+05 | 1.93E-03 |
| KD-22-0224 54E4-6 | 7.56E-10 | 1.23E+06 | 9.27E-04 |
| KD-22-0225 54A8-2 | 3.88E-09 | 5.09E+05 | 1.97E-03 |
| KD-22-0227 58E3-2 | 4.31E-10 | 1.43E+06 | 6.18E-04 |

### Example 27 Construction of anti-PDL1 humanized antibodies

The heavy and light chain variable regions of the anti-PDL1 murine antibody 58E3-2 were humanized to obtain humanized antibody sequences (SEQ ID NO. 40; and SEQ ID NO. 41). Coding sequences of the humanized antibody sequences were linked to the coding sequences of the human IgG1 heavy chain constant region (SEQ ID NO. 42) and human kappa light chain constant region (SEQ ID NO. 18), respectively. The obtained coding genes were cloned into eukaryotic expression vectors, expressed, and an anti-PDL1 humanized antibody was obtained and named "KA-2016 H58E3-2V4-2-hIgG1". Its variable region sequences (SEQ ID NO. 40; and SEQ ID NO. 41) and full-length light chain (SEQ ID NO. 43) are shown below (heavy and light chain CDRs are underlined).
> Heavy chain variable region of anti-PDL1 humanized antibody KA-2016 H58E3-2V4-2-hIgG1 (SEQ ID NO. 40; HCDRs: SEQ ID NOs. 48/51/50): 
QVQLVESGGGLVKPGGSLRLSCAASGFTFSDYGIHWIRQAPGKGLEWVAFISSGSSTIYYADAVKG
RFTISRDNAKNSLYLQMNSLRAEDTAVYYCTRRDFYGSADAMDYWGQGTTVTVSS
> Light chain variable region of anti-PDL1 humanized antibody KA-2016 H58E3-2V4-2-hIgG1 (SEQ ID NO. 41; LCDRs: SEQ ID NOs. 52/26/53): 
DIQLTQSPSTLSASVGDRVTITCSATSSIGYMHWYQQKPGTSPKRWIYDTSKLASGVPSRFSGSGSG
TEYTLTISSLQPDDFATYYCQLWSSDPPYTFGQGTRLEIK
> Human heavy chain CH1 and Fc sequence (SEQ ID NO. 42):
   hIgG1
> Human light chain CL sequence (SEQ ID NO. 18):
   hkappa
> Light chain (SEQ ID NO. 43):

DIQLTQSPSTLSASVGDRVTITCSATSSIGYMHWYQQKPGTSPKRWIYDTSKLASGVPSRFSGSGSG
TEYTLTISSLQPDDFATYYCQLWSSDPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC

### Example 28 Detection of the binding of anti-PDL1 humanized antibodies to PDL1-expressing cells

293T-PDL1 cells were prepared as a cell suspension at a concentration of 1×10⁷ cells/mL in PBS containing 2% FBS.

Flow cytometry was used to detect the binding of the anti-PDL1 humanized antibody KA-2016 H58E3-2V4-2-hIgG1 or positive control antibody Atezolizumab to 293T-PDL1 cells. The experimental procedure followed that described in Example 23. Detection results are shown in Table 26 and Figure 20.

**Table 26. Detection results of the binding of anti-PDL1 humanized antibodies to PDL1-expressing cells**

| Antibody | Atezolizumab | KA-2016 H58E3-2V4-2-hIgG1 | IgG1 irrelevant antibody |
|---|---|---|---|
| EC50 (µg/mL) | 0.05279 | 0.04187 | N/A |
| MFI.Max | 448000 | 474000 | 644 |

### Example 29 Detection of the blocking of PDL1-mFc binding to PD1-expressing cells by anti-PDL1 humanized antibodies

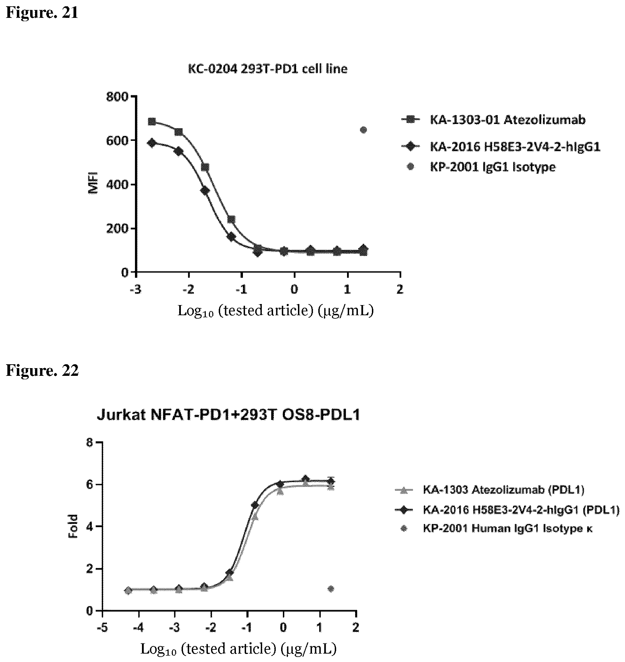

The blocking of PDL1-mFc binding to PD1-expressing cells by anti-PDL1 humanized antibodies was detected. The experimental procedure followed that described in Example 24, in which 50 µL of the serially diluted humanized antibody KA-2016 H58E3-2V4-2-hIgG1 or positive control antibody Atezolizumab and 500 ng of human PDL1-mFc were added per well as a premix, and finally co-incubated with PE conjugated anti-mouse IgG Fc secondary antibody. Detection results are shown in Table 27 and Figure 21.

**Table 27. Detection results of the blocking of PDL1 binding to PD1-expressing cells by anti-PDL1 humanized antibodies**

| Antibody | Atezolizumab | KA-2016 H58E3-2V4-2-hIgG1 | IgG1 irrelevant antibody |
|---|---|---|---|
| IC50 (µg/mL) | 0.0305 | 0.02289 | N/A |

### Example 30 Detection of the blocking of PDL1/PD1 signaling by anti-PDL1 humanized antibodies

The blocking of PDL1/PD1 Signaling by anti-PDL1 humanized antibodies was detected. The experimental procedure followed that described in Example 25. Detection results are shown in Table 28 and Figure 22.

**Table 28. Detection results of the blocking of PDL1/PD1 signaling by anti-PDL1 humanized antibodies**

| Antibody | Atezolizumab | KA-2016 H58E3-2V4-2-hIgG1 | hIgG1 |
|---|---|---|---|
| | | | irrelevant antibody |
| IC50 (nM) | 0.098 | 0.081 | N/A |
| Top | 5.944 | 6.169 | N/A |

### Example 31 Preparation of hybridoma cells secreting anti-EGFR antibodies

Mice constructed in Example 2 were immunized with a fusion protein (referred to as "EGFR-mFc"; Kyinno, Cat No.: KP-1148) of the human EGFR extracellular domain (NCBI Reference Sequence: BC118665.1) and mouse Fc. The experimental procedure followed that described in Example 3. Mice with higher titers were selected, from which sera were collected. Then the mice were dissected, their spleens were taken and spleen cells were isolated. The spleen cells were fused with cultured myeloma cells, and hybridoma cells were obtained.

ELISA plates were coated with EGFR-his (Kyinno, Cat No.: KP-1341). The binding activity of hybridoma cell culture supernatants to the EGFR-his was detected by ELISA, yielding multiple positive hybridoma cell lines secreting anti-EGFR antibodies.

### Example 32 Detection of the binding of hybridoma cell culture supernatants containing anti-EGFR antibodies to EGFR-expressing cells

The gene sequence encoding human EGFR (NCBI Reference Sequence: BC118665.1) was cloned into the PLVX packaging vector (Clontech, virus package mix, Cat No.: 631275), and the recombinant plasmid was transfected into CT26 cells; then, resistant cell lines were selected with puromycin, obtaining CT26 cells stably expressing human EGFR (referred to as "CT26-EGFR cells"; Kyinno, Cat No.: KC-1451). The CT26-EGFR cells were then prepared as a cell suspension at a concentration of 1×10⁷ cells/mL in PBS containing 2% FBS.

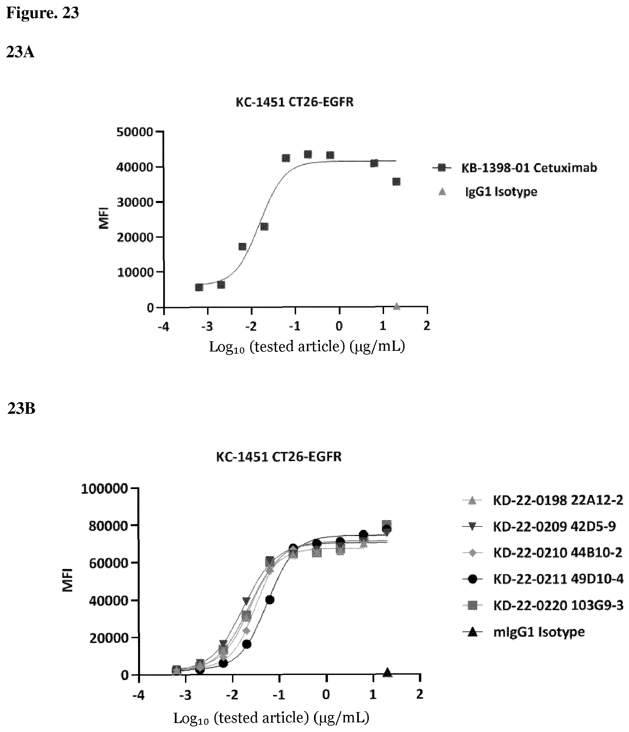

Flow cytometry was used to detect the binding of hybridoma cell culture supernatants containing anti-EGFR antibodies to CT26-EGFR cells. The experimental procedure followed that described in Example 4, differing in the use of Cetuximab (Kyinno, Cat No.: KA-1398), and the addition of 5 µL of PE-conjugated anti-mouse IgG Fc secondary antibody to detect the binding of the hybridoma cell culture supernatants and the addition of 5 µL of Goat anti-human IgG-PE secondary antibody (SouthernBiotech, Cat No.: 2010-09) to detect the binding of the positive control antibody Cetuximab. Detection results are shown in Table 29 and Figure 23.

**Table 29. Detection results of the binding of hybridoma cell culture supernatants containing anti-EGFR antibodies to EGFR-expressing cells**

| Hybridoma cell line | Cetuximab | KD-22-0198 22A12-2 | KD-22-0209 42D5-9 | KD-22-0210 44B 10-2 | KD-22-0211 49D 10-4 | KD-22-0220 103G9-3 | mIgG1 irrelevant antibody |
|---|---|---|---|---|---|---|---|
| EC50 (µg/mL) | 0.01554 | 0.02418 | 0.01733 | 0.03188 | 0.05600 | 0.02256 | N/A |

A murine antibody was obtained from the hybridoma cell line KD-22-0198 22A12-2 and was named "22A12-2". Its variable region sequences are as follows (heavy and light chain CDRs are underlined):
> Heavy chain variable region of anti-EGFR murine antibody 22A12-2 (SEQ ID NO. 44; HCDRs: SEQ ID NOs. 54/55/56): 
EVQLQQSGPELVKPGASVKISCKASGYTFTDYYMNWVKQSHGKSLEWIGDINPNDGGTSYNQKF
KGKATLTVDKSSSTAYMELRSLTSEDSAVYYCAREILYYVTSFYFDYWGQGTTLTVSS
> Light chain variable region of anti-EGFR murine antibody 22A12-2 (SEQ ID NO. 13; LCDRs: SEQ ID NOs. 31/33/32):

QIVLTQSPAIMSASPGEKVTMTCSATSGVSYMHWYQQKSGTSPKRWIYDTSRLASGVPARFSGSGS
GTSYSLTISSMEAEDAATYYCQQWSSNPPYTFGSGTKLEIK

### Example 33 Detection of the blocking of EGFR-Fc binding to EGF by anti-EGFR murine antibodies

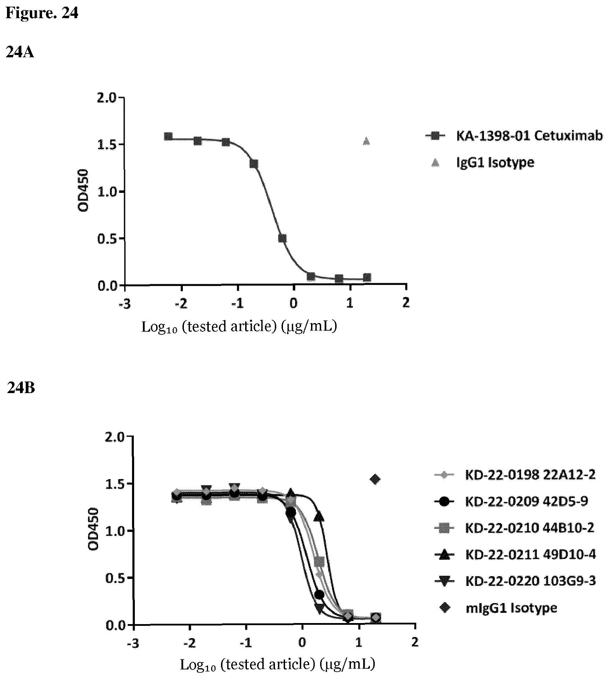

ELISA plates were coated overnight at 4 °C with EGF-His dissolved in PBS (2 µg/mL; Acro, Cat No.: EGF-H52H3), and next day, the plates were blocked for 1 hour at room temperature with blocking buffer (PBS containing 1% BSA). Serially diluted control antibody Cetuximab or hybridoma antibodies were co-incubated with 0.4 µg/mL EGFR-mFc (Kyinno, Cat No.: KP-1148) or EGFR-hFc (Kyinno, Cat No.: KP-1149), and the mixtures were then added to the EGF-His-coated plates and incubated for 1 hour at room temperature. Then the plates were washed with PBS containing 0.5% Tween-20, followed by co-incubation with HRP-conjugated anti-mouse IgG secondary antibody (1:10000 diluted) or with HRP-conjugated anti-human IgG secondary antibody (1:10000 diluted). Then the plates were washed with PBS containing 0.5% Tween-20 again, and HRP substrate was added for color development. Binding signal was detected, and IC50 values were calculated. Detection results are shown in Table 30 and Figure 24.

**Table 30. Detection of blocking of EGFR binding to EGF by anti-EGFR murine antibodies**

| Antibody | Cetuximab | KD-22-0198 22A12-2 | KD-22-0209 42D5-9 | KD-22-0210 44B10-2 | KD-22-0211 49D 10-4 | KD-22-0220 103G9-3 | mIgG1 irrelevant antibody |
|---|---|---|---|---|---|---|---|
| IC50 (µg/mL) | 0.4083 | 1.579 | 1.196 | 1.912 | 2.760 | 0.9592 | N/A |

### Example 34 Detection of the blocking of EGFR signaling by anti-EGFR murine antibodies

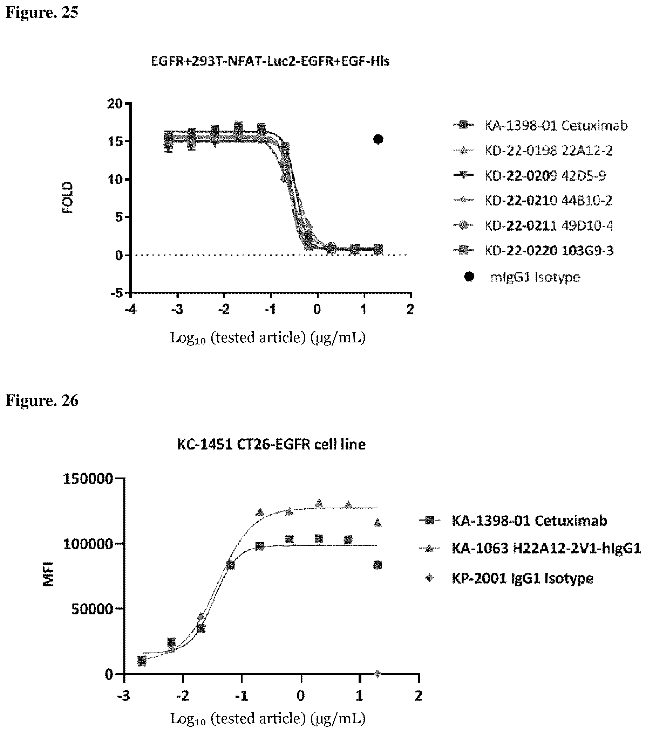

293T-NFAT-Luc2-EGFR cells (Kyinno, Cat No.: KC-2952; expressing luciferase upon EGFR signaling activation) were adjusted to a cell density of 20000 cells/80 µL in RPMI-1640 + 10% FBS medium. 10 µL of the serially diluted positive control antibody Cetuximab or one of the anti-EGFR murine antibodies was added to the plates per well and co-incubated for 30 minutes; and then 10 µL of EGF-His (final concentration: 20 ng/mL; Acro, Cat No.: EGF-H52H3) was added per well to continue overnight culture. Subsequently, 50 µL of Bright-Lite solution (Vazyme, Cat No.: DD1204) was added to each well and luciferase activity was detected. Wells without EGF added served as the background activation control. Fold increase in activation (relative to background) was plotted against antibody concentration, and IC50 values for the anti-EGFR antibodies were calculated. Detection results are shown in Table 31 and Figure 25.

**Table 31. Detection results of the blocking of EGFR signaling by anti-EGFR murine antibodies**

| Antibody | Cetuximab | KD-22-0198 22A12-2 | KD-22-0209 42D5-9 | KD-22-0210 44B 10-2 | KD-22-0211 49D 10-4 | KD-22-0220 103G9-3 | mIgG 1 irrelevant antibody |
|---|---|---|---|---|---|---|---|
| IC50 (µg/mL) | 0.3462 | 0.3735 | 0.2944 | 0.2876 | 0.2586 | 0.2595 | N/A |

### Example 35 Detection of affinity of anti-EGFR murine antibodies for EGFR

ForteBio Octet instrument was used to detect the affinity of anti-EGFR murine antibodies for EGFR. The mobile phases were EGFR-His (Kyinno, Cat No.: KP-1150) which was diluted to concentrations of 200 nM, 100 nM, 50 nM, 25 nM, 12.5 nM, 6.25 nM, and 3.12 nM. Results are shown in Table 32.

**Table 32. Affinity of anti-EGFR murine antibodies for EGFR**

| Antibody | KD (M) | kon(1/Ms) | kdis(1/s) |
|---|---|---|---|
| KD-22-0198 22A12-2 | 1.15E-09 | 9.65E+05 | 1.11E-03 |
| KD-22-0209 42D5-9 | 2.70E-09 | 1.07E+06 | 2.90E-03 |
| KD-22-0210 44B10-2 | 2.03E-09 | 1.15E+06 | 2.34E-03 |
| KD-22-0211 49D10-4 | 1.99E-09 | 1.21E+06 | 2.40E-03 |
| KD-22-0220 103G9-3 | 7.31E-10 | 9.89E+05 | 7.23E-04 |

### Example 36 Construction of anti-EGFR humanized antibodies

The heavy chain variable region of the anti-EGFR murine antibody 22A12-2 was humanized to obtain a humanized antibody sequence (SEQ ID NO. 45); additionally, the light chain variable region of the anti-PD1 humanized antibody provided in Example 9 (SEQ ID NO. 15) was used as the light chain variable region to construct an anti-EGFR humanized antibody. Coding sequences of the humanized antibody sequences were linked to the coding sequences of the human IgG1 heavy chain constant region (SEQ ID NO. 42) and human kappa light chain constant region (SEQ ID NO. 18), respectively. The obtained coding genes were cloned into eukaryotic expression vectors, expressed, and an anti-EGFR humanized antibody was obtained and named "KA-1063 H22A12-2V1-hIgG1". Its variable region sequences (SEQ ID NO. 45; and SEQ ID NO. 15) and full-length light chain (SEQ ID NO. 37) are shown below (heavy and light chain CDRs are underlined):
> Heavy chain variable region of anti-EGFR humanized antibody KA-1063 H22A12-2V1-hIgG1 (SEQ ID NO. 45; HCDRs: SEQ ID NOs. 54/55/56): 
QVQLVQSGAEVKKPGASVKVSCKASGYTFTDYYMNWVRQAPGQGLEWIGDINPNDGGTSYNQ
KFKGRATLTVDKSTSTAYMELSSLRSEDTAVYYCAREILYYVTSFYFDYWGQGTTVTVSS
> Light chain variable region of anti-EGFR humanized antibody KA-1063 H22A12-2V1-hIgG1 (light chain variable region of anti-PD1 humanized antibody KA-1807 H107D12-10V4-hIgG4M; SEQ ID NO. 15; LCDRs: SEQ ID NOs. 31/33/32): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIK
> Human heavy chain CH1 and Fc sequence (SEQ ID NO. 42):
   hIgG1
> Human light chain CL sequence (SEQ ID NO. 18):
   hkappa
> Light chain (SEQ ID NO. 37):

DIQITQSPSSLSASVGDRVTITCSATSGVSYMHWYQQKPGKAPKRLIYDTSRLASGVPSRFSGSGSG

TDYTLTISSLQPEDFATYYCQQWSSNPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC

### Example 37 Detection of the binding of anti-EGFR humanized antibodies to EGFR-expressing cells

The CT26-EGFR cells were prepared as a cell suspension at a concentration of 1×10⁷ cells/mL in PBS containing 2% FBS.

Flow cytometry was used to detect the binding of anti-EGFR humanized antibodies to CT26-EGFR cells. The experimental procedure followed that described in Example 32. Detection results are shown in Table 33 and Figure 26.

**Table 33. Detection results of the binding of anti-EGFR humanized antibodies to EGFR-expressing cells**

| Antibody | Cetuximab | KA-1063 H22A12-2V 1-hIgG1 | IgG1 irrelevant antibody |
|---|---|---|---|
| EC50 (µg/mL) | 0.03348 | 0.03815 | N/A |

### Example 38 Detection of the blocking of EGFR-Fc binding to EGF by anti-EGFR humanized antibodies

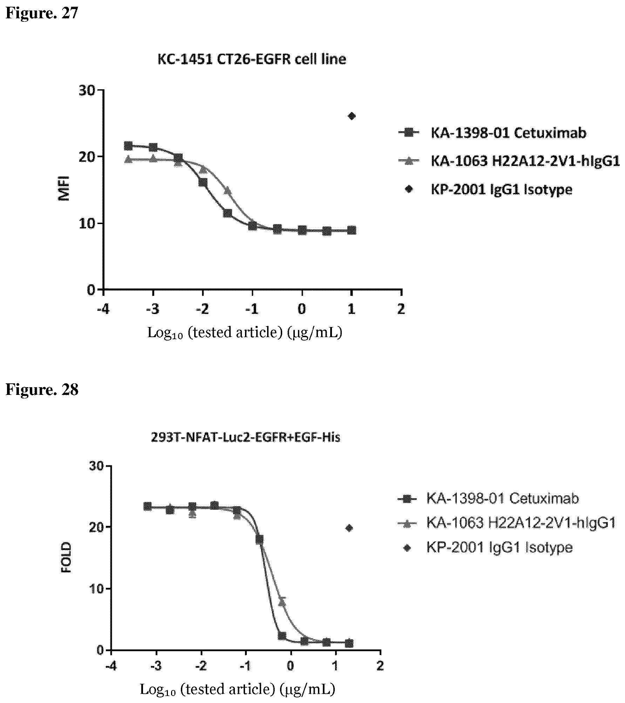

The blocking of EGFR binding to EGF by anti-EGFR humanized antibodies was detected. The experimental procedure followed that described in Example 33, differing in the use of control antibody Cetuximab and humanized antibody KA-1063 H22A12-2V1-hIgG1 serially diluted which were co-incubated with 0.4 µg/mL EGFR-mFc (Kyinno, Cat No.: KP-1148), and finally co-incubated with HRP conjugated anti-mouse IgG secondary antibody. Detection results are shown in Table 34 and Figure 27.

**Table 34. Detection results of the blocking of EGFR binding to EGF by anti-EGFR humanized antibodies**

| Antibody | Cetuximab | KA-1063 H22A12-2V 1-hIgG1 | IgG1 irrelevant antibody |
|---|---|---|---|
| IC50 (µg/mL) | 0.01195 | 0.03566 | N/A |

### Example 39 Detection of the blocking of EGFR signaling by anti-EGFR humanized antibodies

The blocking of EGFR signaling by anti-EGFR humanized antibodies was detected. The experimental procedure followed that described in Example 34. Detection results are shown in Table 35 and Figure 28.

**Table 35. Detection results of the blocking of EGFR signaling by anti-EGFR humanized antibodies**

| Antibody | KA-1398-01 Cetuximab | KA-1063 H22A12-2V1-hIgG1 | IgG1 irrelevant antibody |
|---|---|---|---|
| IC50 (nM) | 0.2793 | 0.3891 | N/A |

### Example 40 Construction of EGFR×PDL1 bispecific antibodies

Coding sequence of the amino acid sequence shown in SEQ ID NO. 15 or SEQ ID NO. 41 was linked to the coding sequence of the human kappa light chain constant region (SEQ ID NO. 18), thereby obtaining common light chain 1 (SEQ ID NO. 37) or common light chain 3 (SEQ ID NO. 43) which would be used for constructing a bispecific antibody.

Coding sequence of the heavy chain variable region of the anti-EGFR humanized antibody was linked to the coding sequence of the following human heavy chain CH1 and Fc sequence ("Knob"), and coding sequence of the heavy chain variable region of the anti-PDL1 humanized antibody was linked to the coding sequence of the following human heavy chain CH1 and Fc sequence ("Hole").

EGFR×PDL1 bispecific antibodies were prepared. The experimental procedure followed that described in Example 15. The domains comprised in the obtained EGFR×PDL1 bispecific antibodies are shown in Table 36.

**Table 36. Bispecific antibodies comprising common light chain 1 or common light chain 3**

| Bispecific antibody | EGFR-targeting heavy chain | | PDL1-targeting heavy chain | | Common light chain | | |
|---|---|---|---|---|---|---|---|
| | Heavy chain variable region | Heavy chain constant region | Heavy chain variable region | Heavy chain constant region | Light chain variable region | Light chain constant region | Light chain |
| KA-2072 H22A12×58E3-CLC01 | SEQ ID NO. 45 | SEQ ID NO. 46 [Knob] | SEQ ID NO. 40 | SEQ ID NO. 47 [Hole] | SEQ ID NO. 15 | SEQ ID NO. 18 | SEQ ID NO. 37 |
| KA-2073 H22A12×58E3-CLC02 | SEQ ID NO. 45 | SEQ ID NO. 46 [ Knob] | SEQ ID NO. 40 | SEQ ID NO. 47 [Hole] | SEQ ID NO. 41 | SEQ ID NO. 18 | SEQ ID NO. 43 |

> Heavy chain variable region of anti-EGFR humanized antibody KA-1063 H22A12-2V1-hIgG1 (SEQ ID NO. 45; HCDRs: SEQ ID NOs. 54/55/56): 
QVQLVQSGAEVKKPGASVKVSCKASGYTFTDYYMNWVRQAPGQGLEWIGDINPNDGGTSYNQ
KFKGRATLTVDKSTSTAYMELSSLRSEDTAVYYCAREILYYVTSFYFDYWGQGTTVTVSS
> Human heavy chain CH1 and Fc sequence ("Knob"; hIgG1; SEQ ID NO. 46): 
ASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPCRDELTKNQVSLWCLVKGFYPSDIAVEW
ESNGQPENNYKTTPPVLDSDGSFFLYSKLTVDKSRWQQGNVFSCSVMHEALHNHYTQKSLSLSPG
> Heavy chain variable region of anti-PDL1 humanized antibody KA-2016 H58E3-2V4-2-hIgG1 (SEQ ID NO. 40; HCDRs: SEQ ID NOs. 48/51/50): QVQLVESGGGLVKPGGSLRLSCAASGFTFSDYGIHWIRQAPGKGLEWVAFISSGSSTIYYADAVKG RFTISRDNAKNSLYLQMNSLRAEDTAVYYCTRRDFYGSADAMDYWGQGTTVTVSS
> Human heavy chain CH1 and Fc sequence ("Hole"; hIgG1; SEQ ID NO. 47): 
ASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVCTLPPSRDELTKNQVSLSCAVKGFYPSDIAVEWE
SNGQPENNYKTTPPVLDSDGSFFLVSKLTVDKSRWQQGNVFSCSVMHEALHNHYTQKSLSLSPG
> Light chain variable region of anti-EGFR humanized antibody KA-1063 H22A12-2V1-hIgG1 (light chain variable region of anti-PD1 humanized antibody KA-1807 H107D12-10V4-hIgG4M; SEQ ID NO. 15; LCDRs: SEQ ID NOs. 31/33/32): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIK
> **Light chain** (SEQ ID NO. 37): 
DIQITQSPSSLSASVGDRVTITCSATSGVSYMHWYQQKPGKAPKRLIYDTSRLASGVPSRFSGSGSG
TDYTLTISSLQPEDFATYYCQQWSSNPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC
> Light chain variable region of anti-PDL1 humanized antibody KA-2016 H58E3-2V4-2-hIgG1 (SEQ ID NO. 41; LCDRs: SEQ ID NOs. 52/26/53): 
DIQLTQSPSTLSASVGDRVTITCSATSSIGYMHWYQQKPGTSPKRWIYDTSKLASGVPSRFSGSGSG
TEYTLTISSLQPDDFATYYCQLWSSDPPYTFGQGTRLEIK
> Light chain (SEQ ID NO. 43):

DIQLTQSPSTLSASVGDRVTITCSATSSIGYMHWYQQKPGTSPKRWIYDTSKLASGVPSRFSGSGSG
TEYTLTISSLQPDDFATYYCQLWSSDPPYTFGQGTRLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQ
GLSSPVTKSFNRGEC

### Example 41 Detection of the binding of EGFR×PDL1 bispecific antibodies having common light chain 1 or common light chain 3 to PDL1-expressing cells

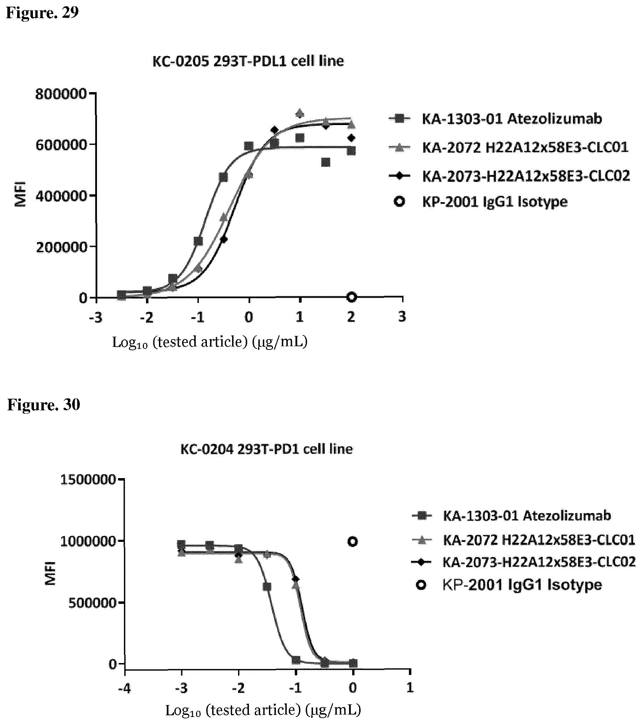

Flow cytometry was used to detect the binding of EGFR×PDL1 bispecific antibodies to PDL1-expressing cells. The experimental procedure followed that described in Example 28. Detection results are shown in Table 37 and Figure 29.

**Table 37. Detection results of the binding of EGFR×PDL1 bispecific antibodies having common light chain 1 or common light chain 3 to PDL1-expressing cells**

| Antibody | Atezolizumab | KA-2072 H22A12×58E3-CLC01 | KA-2073-H22A12×58E3-CLC02 | IgG1 irrelevant antibody |
|---|---|---|---|---|
| EC50 (µg/mL) | 0.1403 | 0.4223 | 0.5213 | N/A |
| MFI.Max | 623000 | 723000 | 716000 | 44.1 |

### Example 42 Detection of the blocking of PDL1-mFc binding to PD1-expressing cells by EGFR×PDL1 bispecific antibodies having common light chain 1 or common light chain 3

The blocking of PDL1-mFc binding to PD1-expressing cells by EGFR×PDL1 bispecific antibodies was detected. The experimental procedure followed that described in Example 29, in which 50 µL of the serially diluted EGFR×PDL1 bispecific antibody KA-2072 H22A12×58E3-CLC01, KA-2073-H22A12 x58E3-CLC0, or positive control antibody Atezolizumab and 1.2 µg/mL of human PDL1-mFc were added per well as a premix, and finally co-incubated with PE conjugated anti-mouse IgG Fc secondary antibody. Detection results are shown in Table 28 and Figure 30.

**Table 38. Detection results of the blocking of PDL1 binding to PD1-expressing cells by EGFR×PDL1 bispecific antibodies**

| Antibody | Atezolizumab | KA-2072 H22A12×58E3-CLC01 | KA-2073 H22A12 ×58E3-CLCO2 | IgG1 irrelevant antibody |
|---|---|---|---|---|
| IC50 (µg/mL) | 0.03796 | 0.1218 | 0.1294 | N/A |

### Example 43 Detection of the blocking of PDL1/PD1 signaling by EGFR×PDL1 bispecific antibodies having common light chain 1 or common light chain 3

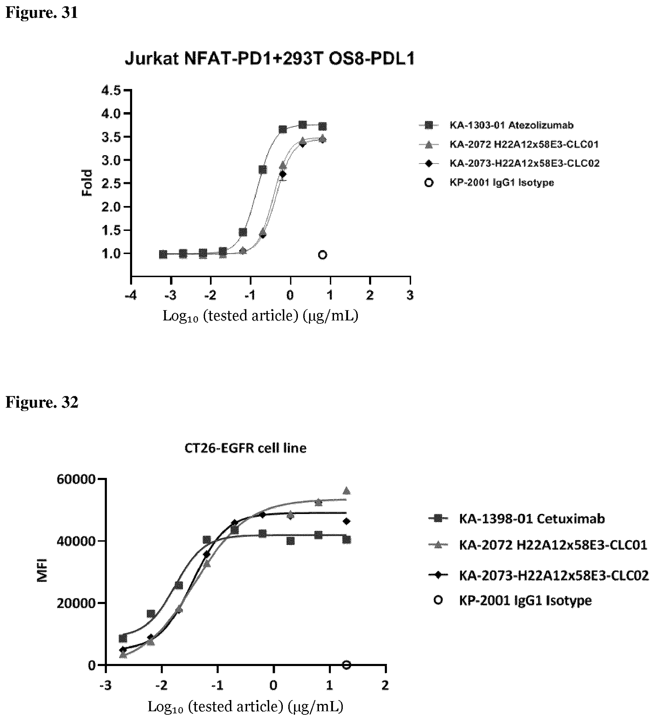

The blocking of PDL1/PD1 signaling by EGFR×PDL1 bispecific antibodies was detected. The experimental procedure followed that described in Example 30. Detection results are shown in Table 39 and Figure 31.

**Table 39. Detection results of the blocking of PDL1/PD1 signaling by EGFR×PDL1 bispecific antibodies having common light chain 1 or common light chain 3**

| Antibody | Atezolizumab | KA-2072 H22A12×58E3-CLC01 | KA-2073 H22A12 ×58E3-CLC02 | IgG1 irrelevant antibody |
|---|---|---|---|---|
| IC50 (nM) | 0.145 | 0.3717 | 0.429 | N/A |
| Top | 3.763 | 3.484 | 3.459 | N/A |

### Example 44 Detection of the binding of EGFR xPDL1 bispecific antibodies having common light chain 1 or common light chain 3 to EGFR-expressing cells

Flow cytometry was used to detect the binding of EGFR×PDL1 bispecific antibodies to CT26-EGFR cells. The experimental procedure followed that described in Example 32, differing in the addition of 5 µL of Goat anti-human IgG-PE secondary antibody (SouthemBiotech, Cat No.: 2010-09). Detection results are shown in Table 40 and Figure 32.

**Table 40. Detection results of the binding of EGFR×PDL1 bispecific antibodies having common light chain 1 or common light chain 3 to EGFR-expressing cells**

| Antibody | Cetuximab | KA-2072 H22A12×58E3-CLC01 | KA-2073 H22A12×58E3-CLC02 | IgG1 irrelevant antibody |
|---|---|---|---|---|
| EC50 (µg/mL) | 0.01714 | 0.0393 | 0.0358 | N/A |
| MFI.Max | 41922 | 56395 | 44339 | 84.2 |

### Example 45 Detection of the blocking of EGF-mFc binding to EGFR-expressing cells by EGFR×PDL1 bispecific antibodies having common light chain 1 or common light chain 3

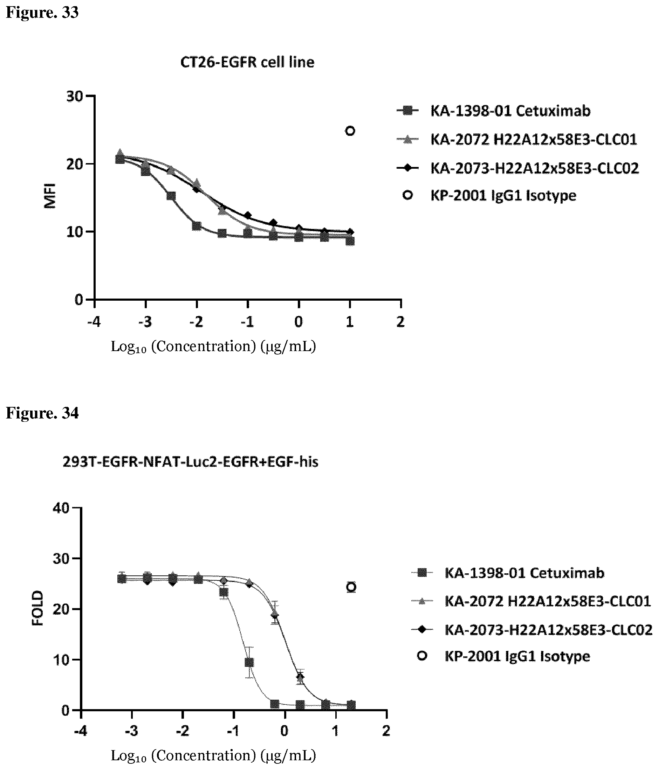

CT26 cells overexpressing human EGFR, i.e., "CT26-EGFR cells", were collected. The EGFR×PDL1 bispecific antibody KA-2072 H22A12×58E3-CLC01, KA-2073-H22A12×58E3-CLC0 or positive control antibody Cetuximab was serially diluted, co-incubated with the CT26-EGFR cells, then co-incubated with 0.05 µg/mL EGF-mFc (Acro, Cat No.: EGF-H525b), finally co-incubated with PE-conjugated anti-mouse IgG Fc secondary antibody (Biolegend, Cat No.: 405307). Flow cytometry was used to detect EGF-mFc binding to the cells. Detection results are shown in Table 41 and Figure 33.

**Table 41. Detection results of the blocking of EGF binding to EGFR-expressing cells by EGFR×PDL1 bispecific antibodies having common light chain 1 or common light chain 3**

| Antibody | Cetuximab | KA-2072 H22A12×58E3-CLC01 | KA-2073 H22A12×58E3-CLC02 | IgG1 irrelevant antibody |
|---|---|---|---|---|
| IC50 (µg/mL) | 0.003156 | 0.0155 | 0.0123 | N/A |

### Example 46 Detection of the blocking of EGFR signaling by EGFR×PDL1 bispecific antibodies having common light chain 1 or common light chain 3

The blocking of EGFR signaling by EGFR×PDL1 bispecific antibodies was detected. The experimental procedure followed that described in Example 34. Detection results are shown in Table 42 and Figure 34.

**Table 42. Detection results of the blocking of EGFR signaling by EGFR×PDL1 bispecific antibodies having common light chain 1 or common light chain 3**

| Antibody | Cetuximab | KA-2072 H22A12×58E3-CLC01 | KA-2073 H22A12×58E3-CLC02 | IgG1 irrelevant antibody |
|---|---|---|---|---|
| IC50 (nM) | 0.152 | 1.013 | 1.046 | N/A |

### Example 47 Detection of affinity of EGFR×PDL1 bispecific antibodies having common light chain 1 or common light chain 3 for EGFR and PDL1

Affinity of the EGFR×PDL1 bispecific antibodies for EGFR and PDL1 was detected. The experimental procedure followed those described in Example 26 and Example 35. Detection results are shown in Table 43.

**Table 43. Detection results of affinity of EGFR ×PDL1 bispecific antibodies having common light chain 1 or common light chain 3 for EGFR and PDL1**

| Antibody | Antigen | KD (M) | kon(1/Ms) | kdis(1/s) |
|---|---|---|---|---|
| KA-1303 Atezolizumab | PDL1-His | 5.39E-10 | 9.52E+05 | 5.13E-04 |
| KA-2072 H22A12×58E3-CLC01 | PDL1-His | 4.64E-10 | 9.18E+05 | 4.26E-04 |
| KA-2073 H22A12×58E3-CLC02 | PDL1-His | 6.02E-10 | 2.09E+06 | 1.26E-03 |
| KA-1398 Cetuximab | EGFR-His | 3.37E-09 | 3.04E+05 | 1.03E-03 |
| KA-2072 H22A12×58E3-CLC01 | EGFR-His | 4.21E-09 | 7.01E+05 | 2.95E-03 |
| KA-2073 H22A12×58E3-CLC02 | EGFR-His | 5.64E-09 | 7.96E+05 | 4.49E-03 |

The above description of the embodiments of the invention is not intended to limit the invention, and those skilled in the art may make various changes and modifications to the invention without departing from the spirit of the invention, which should fall within the scope of the appended claims.

## Claims

1. A polypeptide, the amino acid sequence of which comprises:
(1) the amino acid sequence shown in SEQ ID NO. 25; the amino acid sequence shown in SEQ ID NO. 26; and the amino acid sequence shown in SEQ ID NO. 32; or
(2) the amino acid sequence shown in SEQ ID NO. 31; the amino acid sequence shown in SEQ ID NO. 33; and the amino acid sequence shown in SEQ ID NO. 32; or
(3) the amino acid sequence shown in SEQ ID NO. 52; the amino acid sequence shown in SEQ ID NO. 26; and the amino acid sequence shown in SEQ ID NO. 53.

2. The polypeptide according to claim 1, **characterized in that** the polypeptide comprises the amino acid sequence shown in SEQ ID NO. 2, SEQ ID NO. 13, SEQ ID NO. 15, SEQ ID NO. 16, or SEQ ID NO. 41.

3. The polypeptide according to claim 1 or 2, **characterized in that** the polypeptide comprises the amino acid sequence shown in SEQ ID NO. 35, SEQ ID NO. 36, SEQ ID NO. 37, SEQ ID NO. 38, or SEQ ID NO. 43.

4. An antibody or antigen-binding fragment thereof comprising the polypeptide as defined in any one of claims 1 to 3 as an antibody light chain or light chain variable region;
preferably, the antibody or antigen-binding fragment thereof is an IgG-like antibody having at least two Fab arms and optionally an Fc region.

5. The antibody or antigen-binding fragment thereof according to claim 4, **characterized in that** in its at least one Fab arm, the antibody or antigen-binding fragment thereof comprises the amino acid sequences as defined in any one of (1) to (3) of claim 1 as light chain CDRs; or comprises the amino acid sequence shown in SEQ ID NO. 2, SEQ ID NO. 13, SEQ ID NO. 15, SEQ ID NO. 16, or SEQ ID NO. 41 as a light chain variable region; or
the antibody or antigen-binding fragment thereof comprises the amino acid sequence shown in SEQ ID NO. 35, SEQ ID NO. 36, SEQ ID NO. 37, SEQ ID NO. 38, or SEQ ID NO. 43 as a light chain.

6. The antibody or antigen-binding fragment thereof according to claim 4 or 5, **characterized in that** in its at least one Fab arm, the antibody or antigen-binding fragment thereof comprises heavy chain CDRs (HCDR1, HCDR2, and HCDR3) and light chain CDRs (LCDR1, LCDR2, and LCDR3) respectively as follows:
(1) an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(2) an HCDR1 comprising the amino acid sequence shown in SEQ ID NO. 22, an HCDR2 comprising the amino acid sequence shown in SEQ ID NO. 23, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(3) an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(4) an HCDR1 comprising the amino acid sequence shown in SEQ ID NO. 22, an HCDR2 comprising the amino acid sequence shown in SEQ ID NO. 23, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(5) an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(6) an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(7) an HCDR1 comprising the amino acid sequence shown in SEQ ID NO. 48, an HCDR2 comprising the amino acid sequence shown in SEQ ID NO. 49, and an HCDR3 comprising the amino acid sequence shown in SEQ ID NO. 50; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(8) an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53;
(9) an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(10) an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; or
(11) an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53.

7. The antibody or antigen-binding fragment thereof according to any one of claims 4 to 6, **characterized in that** in its at least one Fab arm, the antibody or antigen-binding fragment thereof comprises a heavy chain variable region and a light chain variable region comprising respectively:
(1) the amino acid sequence shown in SEQ ID NO. 12; and, the amino acid sequence shown in SEQ ID NO. 2;
(2) the amino acid sequence shown in SEQ ID NO. 10; and, the amino acid sequence shown in SEQ ID NO. 2;
(3) the amino acid sequence shown in SEQ ID NO. 12; and, the amino acid sequence shown in SEQ ID NO. 13;
(4) the amino acid sequence shown in SEQ ID NO. 10; and, the amino acid sequence shown in SEQ ID NO. 13;
(5) the amino acid sequence shown in SEQ ID NO. 14; and, the amino acid sequence shown in SEQ ID NO. 15;
(6) the amino acid sequence shown in SEQ ID NO. 19; and, the amino acid sequence shown in SEQ ID NO. 15;
(7) the amino acid sequence shown in SEQ ID NO. 14; and, the amino acid sequence shown in SEQ ID NO. 16;
(8) the amino acid sequence shown in SEQ ID NO. 19; and, the amino acid sequence shown in SEQ ID NO. 16;
(9) the amino acid sequence shown in SEQ ID NO. 39; and, the amino acid sequence shown in SEQ ID NO.2;
(10) the amino acid sequence shown in SEQ ID NO. 40; and, the amino acid sequence shown in SEQ ID NO. 41;
(11) the amino acid sequence shown in SEQ ID NO. 44; and, the amino acid sequence shown in SEQ ID NO. 13;
(12) the amino acid sequence shown in SEQ ID NO. 45; and, the amino acid sequence shown in SEQ ID NO. 15;
(13) the amino acid sequence shown in SEQ ID NO. 40; and, the amino acid sequence shown in SEQ ID NO. 15; or
(14) the amino acid sequence shown in SEQ ID NO. 45; and, the amino acid sequence shown in SEQ ID NO. 41.

8. The antibody or antigen-binding fragment thereof according to any one of claims 4 to 7, **characterized in that** the at least two Fab arms of the antibody or antigen-binding fragment thereof are identical, or are different;
wherein the at least two Fab arms are identical and comprise identical amino acid sequence combination selected from any one of (1) to (11) defined in claim 6 or comprise identical amino acid sequence combination selected from any one of (1) to (14) defined in claim 7.

9. The antibody or antigen-binding fragment thereof according to any one of claims 4 to 8, **characterized in that** the at least two Fab arms are different and comprise different amino acid sequence combinations selected from any one of (1) to (11) defined in claim 6 or comprise different amino acid sequence combinations selected from any one of (1) to (14) defined in claim 7; preferably, the at least two Fab arms of the antibody or antigen-binding fragment thereof comprise identical light chain CDRs sequence combination, or comprise identical light chain variable region sequence.

10. The antibody or antigen-binding fragment thereof according to any one of claims 4 to 9, **characterized in that** the antibody or antigen-binding fragment thereof comprises:
(1) in one Fab arm: an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; and
in another Fab arm: an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32;
(2) in one Fab arm: an HCDR1 comprising the amino acid sequence shown in SEQ ID NO. 28, an HCDR2 comprising the amino acid sequence shown in SEQ ID NO. 29, and an HCDR3 comprising the amino acid sequence shown in SEQ ID NO. 30; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32; and
in another Fab arm: an HCDR1 comprising the amino acid sequence shown in SEQ ID NO. 22, an HCDR2 comprising the amino acid sequence shown in SEQ ID NO. 34, and an HCDR3 comprising the amino acid sequence shown in SEQ ID NO. 24; and, an LCDR1 comprising the amino acid sequence shown in SEQ ID NO. 25, an LCDR2 comprising the amino acid sequence shown in SEQ ID NO. 26, and an LCDR3 comprising the amino acid sequence shown in SEQ ID NO. 32;
(3) in one Fab arm: an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; and
in another Fab arm: an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 31, an LCDR2 comprising the amino acid sequence shown in SEQ ID NO. 33, and an LCDR3 comprising the amino acid sequence shown in SEQ ID NO. 32; or
(4) in one Fab arm: an HCDR1 comprising the amino acid sequence shown in SEQ ID NO. 54, an HCDR2 comprising the amino acid sequence shown in SEQ ID NO. 55, and an HCDR3 comprising the amino acid sequence shown in SEQ ID NO. 56; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53; and
in another Fab arm: an HCDR1 comprising the amino acid sequence shown in SEQ ID NO. 48, an HCDR2 comprising the amino acid sequence shown in SEQ ID NO. 51, and an HCDR3 comprising the amino acid sequence shown in SEQ ID NO. 50; and, an LCDR1 comprising the amino acid sequence shown in SEQ ID NO. 52, an LCDR2 comprising the amino acid sequence shown in SEQ ID NO. 26, and an LCDR3 comprising the amino acid sequence shown in SEQ ID NO. 53.

11. The antibody or antigen-binding fragment thereof according to any one of claims 4 to 10, **characterized in that** the antibody or antigen-binding fragment thereof comprises:
(1) in one Fab arm: a heavy chain variable region comprising the amino acid sequence shown in SEQ ID NO. 14, and a light chain variable region comprising the amino acid sequence shown in SEQ ID NO. 15; and
in another Fab arm: a heavy chain variable region comprising the amino acid sequence shown in SEQ ID NO. 19, and a light chain variable region comprising the amino acid sequence shown in SEQ ID NO. 15;
(2) in one Fab arm: a heavy chain variable region comprising the amino acid sequence shown in SEQ ID NO. 14, and a light chain variable region comprising the amino acid sequence shown in SEQ ID NO. 16; and
in another Fab arm: a heavy chain variable region comprising the amino acid sequence shown in SEQ ID NO. 19, and a light chain variable region comprising the amino acid sequence shown in SEQ ID NO. 16;
(3) in one Fab arm: a heavy chain variable region comprising the amino acid sequence shown in SEQ ID NO. 45, and a light chain variable region comprising the amino acid sequence shown in SEQ ID NO. 15; and
in another Fab arm: a heavy chain variable region comprising the amino acid sequence shown in SEQ ID NO. 40, and a light chain variable region comprising the amino acid sequence shown in SEQ ID NO. 15; or
(4) in one Fab arm: a heavy chain variable region comprising the amino acid sequence shown in SEQ ID NO. 45, and a light chain variable region comprising the amino acid sequence shown in SEQ ID NO. 41; and
in another Fab arm: a heavy chain variable region comprising the amino acid sequence shown in SEQ ID NO. 40, and a light chain variable region comprising the amino acid sequence shown in SEQ ID NO. 41.

12. Use of the polypeptide as defined in any one of claims 1 to 3 in the construction of an antibody.

13. A nucleic acid molecule comprising a nucleotide sequence encoding the polypeptide as defined in any one of claims 1 to 3.
